# EUROPEAN PATENT APPLICATION

(11) **EP 3 974 540 A1**
(43) Date of publication of application: **30.03.2022**
(21) Application number: 20198305.3
(22) Date of filing: 25.09.2020
(51) Int. Cl.: C12Q 1/6886

(54) **METHOD FOR PREDICTING IMMUNOTHERAPY RESISTANCE**

(71) Applicant: Koninklijke Philips N.V., 5656 AG Eindhoven (NL)
(72) Inventor: VAN DE STOLPE, Anja, 5656 AE Eindhoven (NL); WESSELING-ROZENDAAL, Yvonne, 5656 AE Eindhoven (NL)
(74) Representative: Philips Intellectual Property & Standards

(57) **Abstract**

The present invention relates to a method for determining the immunosuppressive state of a subject having a tumor by analyzing the Notch cellular signaling pathways activity. The method can be used in assessing whether immune checkpoint inhibitors are a suitable treatment option for the subject. The invention further relates kits and uses thereof.

## Description

### FIELD OF THE INVENTION

The present invention relates to the field of diagnostics, more particularly to the field of diagnosing or predicting the response of a subject having a tumor to treatment with an immune checkpoint inhibitor. The invention further relates to methods for identifying or distinguishing specific types of CD4+ T cells. The invention also relates to use of checkpoint inhibitors in the treatment of cancer, wherein suitability of the use is first determined with a diagnostic method. Lastly the invention relates to kits and uses thereof suitable for the methods and uses described here.

### BACKGROUND OF THE INVENTION

Infiltration of cancer tissue by a variety of immune cells is necessary to mount an adequate immune response against the cancer cells. During the past decades, evidence has been accumulating that cancer tissue can be highly successful in creating an immune-tolerant environment, which interferes with the appropriate anti-cancer immune response of tumor infiltrating T cells. Checkpoint inhibitor immunotherapy against cytotoxic T lymphocyte antigen-4 (CTLA-4) Programmed Death-1 (PD-1), Programmed Death-ligand 1 (PD-L1), lymphocyte activation gene-3 (LAG-3), T cell immunoglobulin and mucin-domain containing-3 (TIM-3), T cell immunoglobulin and ITIM domain (TIGIT) and V-domain Ig suppressor of T cell activation (VISTA) aims at restoring the effector function of CD8+ cytotoxic T cells, and when successful, has been shown to have the potential of being a curative treatment. An increasing number of immunotherapy drugs that aim at re-activating the immune response in such a targeted manner is in development. Unfortunately, only a limited percentage of patients respond to this type of immunotherapy [1]. Assays to predict response, such as PD-1/PD-L1 and CD4+/CD8+ immunohistochemistry (IHC) staining measurements have proven to be not sufficiently reliable in predicting response in the individual patient. Consequently, there is a high need for assays to predict therapy response and to assess, as soon as possible, whether the installed therapy is effective [1]-[4]. The above problems, among others, are overcome by the methods, uses and products of the invention as detailed by the appended claims.

### SUMMARY OF THE INVENTION

In a first aspect, the invention relates to a method for in vitro or ex vivo diagnosing an immunosuppressed state in a subject having a tumor, based on cells in a blood sample obtained from said subject, the method comprising
- inferring the Notch cellular signaling pathway activity in the blood sample based on the determined expression levels of three or more target genes in the blood sample obtained from the subject, and
- determining the presence of activated Treg cells in the blood sample obtained from the subject based on the inferred Notch cellular signaling pathway activity, and
- diagnosing the subject in an immunosuppressed state when Treg cells which are in the immune suppressive state are determined to be present in the blood sample,
wherein a high inferred Notch cellular signaling pathway activity in the blood sample obtained from the subject is indicative for the presence of activated Treg cells,
wherein the subject is diagnosed to have an immunosuppressed state when activated Treg cells are present.

In a second aspect, the invention relates to a method for monitoring a treatment response for a subject having a tumor, the subject receiving checkpoint inhibitors, wherein the method comprises diagnosing the immunosuppressive state of the subject as described in the first aspect of the invention,
preferably the method further comprising changing the treatment of the subject from treatment with checkpoint inhibitors to treatment with an alternative treatment option once the subject is diagnosed to be in the immunosuppressive state.

In a third aspect the invention relates to an immune checkpoint inhibitor for use in the treatment of cancer in a subject diagnosed to be not in an immunosuppressed state.

In a fourth aspect the invention relates to a method for characterizing a T cell based on inferred cellular signaling pathway activities,
wherein the T cell is determined to be a naive T cell if the inferred pathway activity for FOXO is high, for NFkB is low, for JAK-STAT1/2 is low, for JAK-STAT3 is low, for TGFbeta is intermediate and for Notch is low;
wherein the T cell is determined to be an activated T cell if the inferred pathway activity for FOXO is low, for NFkB is intermediate, for JAK-STAT1/2 is intermediate, for JAK-STAT3 is intermediate, for TGFbeta is low and for Notch is low;
wherein the T cell is determined to be a T-helper 1 cell if the inferred pathway activity for FOXO is low, for NFkB is high, for JAK-STAT1/2 is intermediate, for JAK-STAT3 is intermediate, for TGFbeta is low and for Notch is low;
wherein the T cell is determined to be a T-helper 2 cell if the inferred pathway activity for FOXO is intermediate, for NFkB is low, for JAK-STAT1/2 is low, for JAK-STAT3 is intermediate, for TGFbeta is low and for Notch is low;
wherein the T cell is determined to be a resting state Treg cell if the inferred pathway activity for FOXO is intermediate, for NFkB is low, for JAK-STAT1/2 is intermediate, for JAK-STAT3 is intermediate, for TGFbeta is low and for Notch is intermediate;
wherein the T cell is determined to be an activated Treg cell if the inferred pathway activity for FOXO is high, for NFkB is high, for JAK-STAT1/2 is low, for JAK-STAT3 is high, for TGFbeta is intermediate and for Notch is high.

In a fifth aspect the invention relates to a kit of parts comprising primers and probes for detecting the expression levels of three or more target genes of the Notch and JAK-STAT3 cellular signaling pathways, and optionally for detecting the expression levels of three or more target genes of one or more cellular signaling pathway selected from FOXO, NFkB, JAK-STAT1/2 and TGFbeta, wherein
the three or more Notch target genes are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target genes are selected from the group consisting of: DTX1, HES1, HES4, HES5, HEY2, MYC, NRARP, and PTCRA, and one or more Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIFIA, HSP90AA1, HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1;
the three or more PI3K/FOXO target genes are selected from the group consisting of AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXI1, NOS3, PCK1, POMC, PPARGCIA, PRDX3, RBL2, SOD2 and TNFSF10, optionally further comprising at least one target gene selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally further comprising at least one target gene selected from the group comprising or consisting of: ATG14, BIRC5, IGFBP1, KLF2, KLF4, MYOD1, PDK4, RAG1, RAG2, SESN1, SIRT1, STK11 and TXNIP, more preferably the three or more PI3K/FOXO target genes are selected from the group consisting of AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCK1, POMC, PPARGCIA, PRDX3, RBL2, SOD2 and TNFSF10;
the three or more NFkB target genes are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;
the three or more JAK-STAT1/2 target genes are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E;
the three or more TGFbeta target genes are selected from the group consisting of ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7.

In a sixth aspect the invention relates to the use of the kit according to the fifth aspect of the invention in the in vitro or ex vivo diagnosis of an immunosuppressed state in a subject having a tumor, wherein the diagnosis is based on cells in a blood sample.

### DETAILED DESCRIPTION OF THE INVENTION

In the tumor infiltrate, CD4+ T cells play an important role in activating adaptive immune responses, and when reverted to an immune suppressed state they impair the anti-cancer immune response. Their functional state is regulated by signal transduction pathways, e.g. the PI3K, JAK-STAT1/2, JAK-STAT3, NFκB, TGFβ, and Notch pathways. Recently, assays have been developed which enable quantitative measurement of the activity of these signal transduction pathways in tissue and blood samples.

These signaling pathway assays were used to in vitro characterize resting (naive), immune-activated and immunotolerant CD4+ T cells, as well as CD4+ T-helper 1 (Th1), T-helper 2 (Th2), and regulatory T (Treg) cells, in terms of the activity of signaling pathways. It is here demonstrated that the identified pathway activity profiles can be used in elucidating the mechanism leading to cancer-induced immunotolerance, and to investigate the type and the functional state of CD4+ T cells in blood, lymph node, and tumor infiltrate (TIL) samples from cancer patients [5].

The present invention builds further on these developments to provide a method which enables to diagnose the immunosuppressive state of a subject.

Therefore in a first aspect the invention relates to a method for *in vitro* or *ex vivo* diagnosing an immunosuppressed state in a subject having a tumor, based on cells in a blood sample obtained from said subject, the method comprising
- inferring the Notch cellular signaling pathway activity in the blood sample based on the determined expression levels of three or more target genes in the blood sample obtained from the subject, and
- determining the presence of activated Treg cells in the blood sample obtained from the subject based on the inferred Notch cellular signaling pathway activity, and
- diagnosing the subject in an immunosuppressed state when Treg cells which are in the immune suppressive state are determined to be present in the blood sample,
wherein a high inferred Notch cellular signaling pathway activity in the blood sample obtained from the subject is indicative for the presence of activated Treg cells, wherein the subject is diagnosed to have an immunosuppressed state when activated Treg cells are present.

Preferably the method is performed when treatment of the subject with checkpoint inhibitors is being considered or when the subject is currently being treated with immune checkpoint inhibitors.

The invention is based on the finding that Notch activity can be quantitatively determined in cells in a blood sample, and the finding that activated Treg cells have high Notch signaling and result in the patient being in an immunosuppressed state.

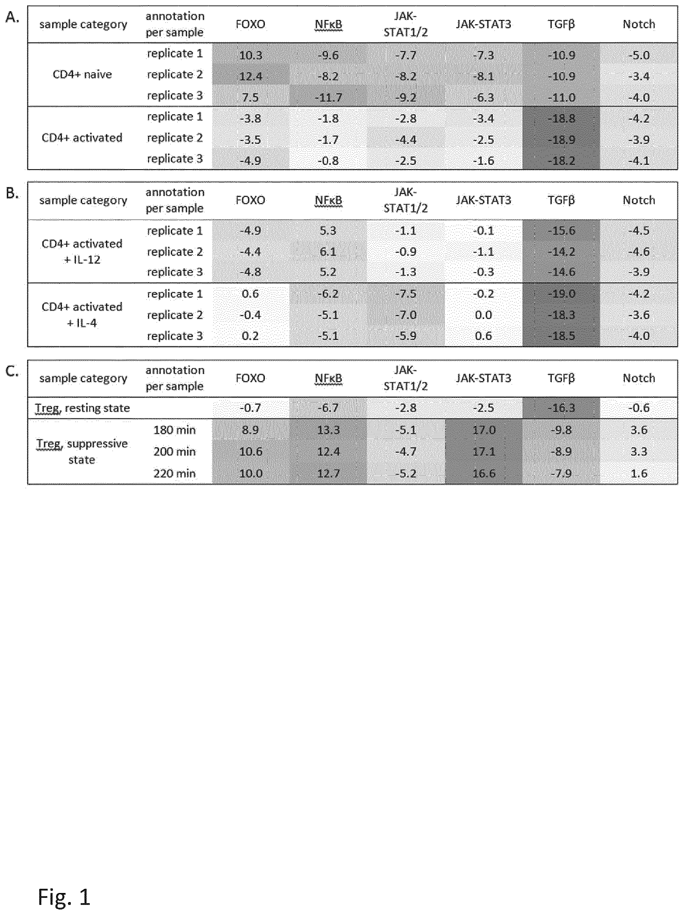

It was found that Notch activity can be used to distinguish activated Treg cells from naive T cells, activated T cells, T-helper 1 cells, T-helper 2 cells and resting Treg cells (see examples, particularly the data represented in Fig. 1). It is well established that activated Treg cells have an immune suppressive function. It has recently been described that activated Treg cells play an important role in immune suppression in cancer tissue [6] and mediate resistance of tumors to treatment with immune checkpoint inhibitors [7]. Ohue and Nishikawa [6] provide a substantive review of the current state of knowledge how activated Treg cells (effector Treg cells) deploy a multitude of mechanisms to suppress immune functions. Tanaka and Sakaguchi [8] give a detailed review on, among others, the role of Treg cells in tumor mediated immunosuppression. In brief, many have shown that a large number of Treg cells are infiltrating into tumor tissues in cancer patients, and that high frequency of tumor-infiltrating Foxp3+ Treg cells, in particular, a low ratio of CD8+ T cells to Treg cells in tumor tissues, is significantly correlated with poor prognosis. Further healthy individuals harbor T cells recognizing tumor-associated antigens as well as antigenically normal self-antigens and their T-cell receptor (TCR) affinities for self-antigens are largely similar to those for non-self-antigens such as viruses. Therefore a considerable number of tumor-reactive T cells and potentially pathogenic self-reactive T cells have escaped thymic negative selection, persisting in the periphery, and Treg cells are actively suppressing their activation and expansion in the periphery including tumor tissues.

Saleh and Elkord [7] provide a comprehensive review detailing how achieving durable response rates in some cancer patients remains a challenge as a result of acquired resistance and tumor immune evasion. Resistance may be driven by the cellular and molecular suppressive network within the tumor microenvironment or due to the loss of tumor antigens. The contribution of the immunosuppressive cellular and molecular tumor network to the development of acquired resistance, through among others Tregs, against cancer immunotherapies are described. The review among others details that a positive correlation between increased numbers of intratumoral Treg and poor prognosis has been reported in patients, and intratumoral Tregs are highly activated and immunosuppressive; they inhibit the activation of cytotoxic CD8+ T cells and effector CD4+ T cells, and limit their proliferation and survival via different means. Saleh and Elkord [9] further provide an extensive review of Tregs in tumor mediated immune checkpoint inhibitor resistance.

The method of the invention is therefore particularly suitable for identifying subjects having a tumor which would benefit from treatment with immune checkpoint inhibitors (when the subject is found not to be in an immunosuppressed state, so no cells with high Notch cellular signaling pathway activity present in the blood sample) and subjects having a tumor which would not benefit from treatment with an immune checkpoint inhibitor (when the subject is found to be in an immunosuppressed state, so cells with high Notch cellular signaling pathway activity present in the blood sample). Therefore, the method is preferably performed based on cells in a blood sample obtained from a subject having a tumor, which subject is being considered for treatment with an immune checkpoint inhibitor or which subject is currently undergoing treatment with an immune checkpoint inhibitor.

Here it is demonstrated for the first time that the Notch cellular signaling pathway activity can be quantitatively determined on cells from a blood sample, and that this pathway activity can be used to identify activated Treg cells in a blood sample.

It is envisioned that the method may be performed on whole blood or a subset of cells present in blood. Therefore, when used herein "cells in a blood sample" is used interchangeably with the term "blood sample" and may refer to a whole blood sample, or cells obtained from the whole blood sample by any means of separation, and may thus refer to a subset of cell, e.g. nucleated cells or peripheral blood mononuclear cells (PBMCs), or a specific subset of cells isolated from PBMCs. It may be particularly preferred to isolate T cells, or even more preferred CD4+ T cells, from the whole blood sample. Therefore, in an embodiment, the cells in the blood sample are T cells, preferably wherein the cells in the blood sample are isolated T cells, more preferably wherein the cells in the blood sample are isolated CD4+ T cells. Methods of cells, e.g. PBMCs or T cells are known to the skilled person, e.g. by using centrifugation on a concentration gradient. When used herein, "blood sample" or "cells in a blood sample" may also refer to tumor infiltrate (TIL) samples from cancer patients.

For example:
To isolate PBMCs, whole blood, diluted with PBS, is gently layered over an equal volume of Ficoll in a Falcon tube and centrifuged for 30-40 minutes at 400-500 g without brake. Four layers will form, each containing different cell types-the uppermost layer will contain plasma, which can be removed by pipetting. The second layer will contain PBMCs and is a characteristically white and cloudy "blanket." These cells can be gently removed using a Pasteur pipette and added to warm medium or PBS to wash off any remaining platelets. However other methods to isolate PBMCs are known to the person skilled in the art.

T cells may be isolated from PBMCs for example using Dynabeads^{®} (ThermoFisher Scientific), using protocols described on their website or included with the product. Briefly: A mixture of mouse IgG antibodies against the non-T cells is added to the starting sample. Depletion Dynabeads^{®} are added and will bind to the antibody-labeled cells during a short incubation. The bead-bound cells are subsequently separated on a magnet and discarded. The remaining, untouched human T cells can be used for any application. However the skilled person is aware of and familiar with other methods for isolating T cells from PBMCs.

CD4+ T cells may be isolated for example using a CD4+ T cell enrichment column (R&D Ssytems), using protocols described on their website or included with the product. Briefly: Using high affinity negative selection of CD4+ T lymphocytes from preparations of peripheral blood mononuclear cells (PBMC) or splenocytes, a leukocyte suspension is incubated with a mixture of monoclonal antibodies and is then loaded onto the T Cell Subset Column. B cells and non-selected T cell subsets are tagged by the antibody cocktail and bind to the anti-immunogloblin coated glass beads. Monocytes bind to the column resin via interactions with their Fc receptors. The highly enriched cell preparation in the column eluate is then available for a variety of applications including tissue culture, immune status monitoring, and flow cytometry. However the skilled person is aware of and familiar with other methods for isolating CD4+ T cells from PBMCs.

Alternatively Treg cells can be identified and isolated using known flow cytometry and FACS techniques.

Most humoral responses cannot prevent tumor growth. However, effector cells, such as T cells, macrophages, and natural killer cells, have relatively effective tumoricidal abilities. Effector cell activity is induced by cells that present tumor-specific antigens (TSAs) or tumor-associated antigens (TAAs) on their surface (these cells are called antigen-presenting cells) and is supported by cytokines (e.g., interleukins, interferons). Despite the activity of effector cells, host immunoreactivity may fail to control tumor occurrence and growth. The T cell is the primary cell responsible for direct recognition and killing of tumor cells. T cells carry out immunologic surveillance, then proliferate and destroy newly transformed tumor cells after recognizing TAAs. The T-cell response to tumors is modulated by other cells of the immune system; some cells require the presence of humoral antibodies directed against the tumor cells (antibody-dependent cellular cytotoxicity) to initiate the interactions that lead to the death of tumor cells. In contrast, suppressor T cells inhibit the immune response against tumors.

As described above and in the literature references cited therein, tumors have been known to suppress the immune response through activated Treg cells, allowing evasion or suppression of the tumor against the T cell response. Furthermore, Treg cells may also mediate resistance against immune checkpoint inhibitors.

Therefore when used herein, an immunosuppressed state refers to decreased immune response to a tumor in a subject. The immunosuppressed state may be induced by the tumor or may be caused by other factors influencing the immune system such as but not limited to immune modulating diseases and conditions. An immunosuppressed state when used herein indicates a decreased response or tolerance to immune checkpoint inhibitors in inducing an immune response to the tumor. A decreased response or tolerance to immune checkpoint inhibitors indicates a state in the immune system where administering immune checkpoint inhibitors does not or insufficiently trigger an immune response to the tumor. Because the method aims to detect an immunosuppressed state in a subject where tumor induced resistance to immune checkpoint inhibitors takes place, the term "immunosuppressed state" when used herein may also refer to immune checkpoint inhibitor resistance, or tumor induced immune checkpoint inhibitor resistance.

Therefore when used herein, "diagnosing an immunosuppressed state in a subject" refers to a method to distinguish between subjects having a tumor who would, when administered an immune checkpoint inhibitor, display an immune response to the tumor (referred to as "no immunosuppressed state") and subjects having a tumor who would, when administered an immune checkpoint inhibitor, display no or a very limited immune response (preferably no immune response) to the tumor (referred to as: "immunosuppressed state").

The methods described herein are preferably performed *in vitro* or *ex vivo,* on cells in a blood sample obtained from a subject having a tumor. However it may be desirable to include a step of withdrawing blood from the subject and optionally processing the blood (e.g. by isolating a subset of cells from the blood, such as PBMCs, T cells or CD4+ T cells). The method is based on the principle that signaling pathway activities may be determined based on the expression levels of target genes. Target gene expression levels may for example be based on mRNA expression levels of said target genes, which can be quantitatively determined using techniques such as qPCR or microarray technology. Therefore the method may further comprise a step of determining the expression levels of the target genes of the cellular signaling pathways in the cells in the blood sample. More preferably therefore the method includes a step of isolating mRNA from the cells in the blood sample, in order to determine the expression levels.

According to a preferred embodiment of the invention, the immunosuppressive state is determined based on evaluating a calibrated mathematical model relating the activity or activities of the signaling pathway(s) in the cells in the blood sample to the immunosuppressive state. This model may be programmed to interpret the combination of pathway activities so as to determine the immunosuppressive state of the subject to be diagnosed. In particular, the determination of the immunosuppressive state comprises (i) receiving activity of the respective signaling pathway(s) in the cells of the blood sample of the subject to be diagnosed, (ii) determining the immunosuppressive state of said subject, the determining being based on evaluating a calibrated mathematical model relating the activity of the respective signaling pathway(s) to the immunosuppressive state.

The calibrated mathematical pathway model is preferably a centroid or a linear model, or a Bayesian network model based on conditional probabilities. For example, the calibrated mathematical pathway model may be a probabilistic model, preferably a Bayesian network model, based on conditional probabilities relating immunosuppressive state and the activity or activities of the signaling pathway(s), or the calibrated mathematical pathway model may be based on one or more linear combination(s) of the activity or activities of the signaling pathway(s).

When used herein, Treg cells refer to regulatory T cells. Regulatory T cells, also known as suppressor T cells, are a subpopulation of T cells that, in a normal state, modulate the immune system, maintain tolerance to self-antigens, and prevent autoimmune disease. Tregs express the biomarkers CD4, FOXP3, and CD25 and are thought to be derived from the same lineage as naive CD4 cells. Because effector T cells also express CD4 and CD25, Tregs are very difficult to effectively discern from effector CD4+ T cells. As described herein below, based on the Notch cellular signaling pathway activity, activated Treg cells can be identified. It was found that high Notch signaling is specifically associated with activated Treg cells, and therefore Notch signaling can be used to effectively distinguish activated Treg cells from other CD4+ T cells. The difference in Notch cellular signaling pathway activity is significant but it should be noted that activated Treg cells do not display an extremely strong Notch signaling pathway activity. Therefore it is preferred that the method is based on a quantitative method for determining cellular signaling pathway activity, preferably a method as described herein, in order to distinguish between moderate but significant differences in Notch cellular signaling pathway activity. Because Notch signaling in blood may be highly specific to activated Treg cells, it is envisioned that activated Tregs may also be detected in e.g. whole blood or PBMCs, or T cells isolated from whole blood, and that it thus not necessarily required to isolate CD4+ T cells from blood first, although the latter may be preferred.

As demonstrated by the data presented in Fig. 1, among CD4+ T cells, a high JAK-STAT3 cellular signaling pathway activity is also specific for activated Treg cells, however it is noted that in whole blood other cells (non CD4+ T cells) are known to be present with elevated JAK-STAT3 cellular signaling pathway activity. Therefore, activated Treg cells are preferably not identified based on JAK-STAT3 cellular signaling pathway alone as this may result in false positives. It is however anticipated that the accuracy of identification or detection of activated Treg cells may be increased by combining information of the Notch and the JAK-STAT3 cellular signaling pathway activities. Therefore, in an embodiment of the invention, additionally the JAK-STAT3 cellular signaling pathway is inferred in the cells in the blood sample based on the determined expression levels of three or more target genes, and wherein the presence of Treg cells which are activated is determined based on the combined inferred Notch and JAK-STAT3 cellular signaling pathway activities.

By using a calibrated mathematical model to relate the gene expression levels to a cellular signaling pathway activity, a numerical value can be assigned to the pathway activity. Depending on the model, this value can for example be normalized to result in a value from 0 to 100, where 0 is no pathway activity and 100 is theoretical maximum pathway activity. Alternatively, the value may be normalized such that the average value is 0 and thus decreased pathway activity is represented by a negative value and increased pathway activity is represented by a positive value. It is understood that the values obtained using such model are dependent on the model used, and do not represent absolute values. Therefore, the same model should preferably be used for calibrating, determining reference values and when used in the method of the invention, so that it allows comparison of the obtained numerical values for pathway activity.

Therefore, the numerical value obtained for a pathway activity in a sample may be compared to the numerical value obtained for that pathway activity in a reference sample. By performing such comparison a statement can be made about the pathway activity in the sample (e.g. cell in a blood sample from a subject with a tumor, wherein the subject is in the immunosuppressed state) relative to the pathway activity determined in the reference sample (e.g. cell in a blood sample from a subject with a tumor, wherein the subject is not in the immunosuppressed state). Based on the numerical value a statement can be made about the pathway activity in the sample with respect to the pathway activity in the reference sample, e.g. whether the pathway activity in the sample is higher or lower, in correspondence with the numerical value obtained for the pathway activity. E.g. the Notch cellular signaling pathway activity can be determined in cells in a blood sample from a subject having a tumor. Since it is determined by the inventors that the Notch cellular signaling pathway activity is high in activated Treg cells, this can bet set as a baseline numerical value representing an active pathway, i.e. the reference value. By determining the numerical value for the Notch cellular signaling pathway activity in the blood sample of a subject with a tumor, the numerical value representing that pathway activity can be compared with the reference value, and it can be determined whether the pathway activity is lower or equal (or higher) compared to the reference based on the numerical values. The comparison may be made with multiple reference samples to allow for more accurate results and statistics to be performed. Preferably, a reference sample is used with a known immunosuppressive state in a comparison. More preferably, reference samples are used from one or more subjects that are in the immunosuppressive state and one or more subjects that are not in the immunosuppressive state.

Alternatively, a reference sample can be used to set a baseline pathway activity, allowing further comparison of the pathway activity. For example, the average and standard deviation can be calculated which can be used to calculate values for active Treg cells, and define a threshold when the pathway activity is statistically similar to the reference, and Notch signaling is thus considered high. Alternatively, a reference can be used with a known state (e.g. immunosuppressive state or not immunosuppressive state) and used in the comparison of the pathway activity/activities.

Therefore, in a preferred embodiment the pathway activity is determined to be high or low when the obtained numerical value for the pathway activity differs with at least one standard deviation from the numerical value obtained for the pathway activity in the reference sample. When the value is within the range of one standard deviation it is said to be equal or comparable to the pathway activity in the reference sample. Thus if the reference sample is activated Treg cells and thus have high Notch signaling activity, and if the measured Notch signaling activity is within one standard deviation of the reference value it is considered high as well. If the measured value is more than one, preferably more than two standard deviations lower, the activity is considered low. It is understood that the threshold may also be set higher, for example 2 times the standard deviation or even 3 times the standard deviation. It is further understood that the threshold may be determined using alternative ways, e.g. statistical methods, to determine a significant deviation from the reference value. Similarly, activity may be divided in low, intermediate and high, by first setting an intermediate activity based on a reference with known intermediate activity for the signaling pathway, and setting a threshold for activities which are considered high or low as described above.

It is understood that the reference value for a certain pathway activity in a sample in principle only needs to be determined once. Therefore, the step of determining a reference pathway activity is not an active step in the methods of the invention, as a predetermined reference pathway activity can be used.

For the purpose of the invention determining the expression levels of the target genes based on the extracted RNA may be a part of the method, meaning the method includes the step of determining the expression levels of the target genes on RNA extracted from the cells in the blood sample obtained from the subject using methods known to the skilled person or described herein. The method may further include the step of obtaining a blood sample from the subject in order to extract the RNA. Alternatively, the expression levels may have been determined separately and the determining step (of the expression levels of the target genes) is not an active step in the method of the invention. In such case the expression levels are provided as an input value, e.g. a relative expression level in reference to one or more control gene expression levels.

When used herein, "expression level" refers to a quantification of the number of mRNA copies transcribed from a gene. Generally this number will not be an absolute value but a relative value, and therefore is preferably normalized for example in reference to the expression of one or more housekeeping genes. Housekeeping genes are genes which are assumed to have constant expression levels independent of cell type and/or functional status of the cell (i.e. from a diseased or healthy subject), and therefore can be used to normalize experimentally determined relative expression levels. Housekeeping genes are generally known to the skilled person, non-limiting examples of housekeeping genes that may be used for normalization are beta-actin, glyceraldehyde-3-phosphate dehydrogenase (GAPDH) and Transcription factor IID TATA binding protein (TBP).

Therefore the phrase "expression level of a target gene" denotes a value that is representative of the amount, e.g. a concentration, of a target gene present in a sample. This value may be based on the amount of a transcription product of the target gene (e.g. mRNA) or a translation product thereof (e.g. protein). Preferably, the expression level is based on the amount of mRNA formed from the target gene. In order to determine the expression level, techniques such as qPCR, multiple qPCR, multiplexed qPCR, ddPCR, RNAseq, RNA expression array or mass spectrometry may be used. For example, a gene expression microarray, e.g. Affymetrix microarray, or RNA sequencing methods, like an Illumina sequencer, can be used.

Sets of cellular signaling pathway target genes whose expression levels are preferably analyzed have been identified, alternatively methods for identifying suitable target genes are described herein. For use to determine pathway activity, for example by a mathematical model, three or more, for example, three, four, five, six, seven, eight, nine, ten, eleven, twelve or more, target genes from each assessed cellular signaling pathway can be analyzed to determine pathway activities.

In an embodiment the signaling pathway measurements are performed using qPCR, multiple qPCR, multiplexed qPCR, ddPCR, RNAseq, RNA expression array or mass spectrometry. For example, a gene expression microarray data, e.g. Affymetrix microarray, or RNA sequencing methods, like an Illumina sequencer, can be used.

The term "subject", as used herein refers to any living being. In some embodiments, the subject is an animal, preferably a mammal. In certain embodiments, the subject is a human being, such as a medical subject. Although the invention is not necessarily limited to a particular group of subjects, it will be apparent that a subject having a tumor profits the most form the invention described herein.

The terms "pathway", "signal transduction pathway", "signaling pathway" and "cellular signaling pathway" are used interchangeably herein.

An "activity of a signaling pathway" may refer to the activity of a signaling pathway associated transcription factor (TF) element in the sample, the TF element controlling transcription of target genes, in driving the target genes to expression, i.e., the speed by which the target genes are transcribed, e.g. in terms of high activity (i.e. high speed) or low activity (i.e. low speed), or other dimensions, such as levels, values or the like related to such activity (e.g. speed). Accordingly, for the purposes of the present invention, the term "activity", as used herein, is also meant to refer to an activity level that may be obtained as an intermediate result during "pathway analysis" as described herein.

The term "transcription factor element" (TF element), as used herein, preferably refers to an intermediate or precursor protein or protein complex of the active transcription factor, or an active transcription factor protein or protein complex which controls the specified target gene expression. For example, the protein complex may contain at least the intracellular domain of one of the respective signaling pathway proteins, with one or more co-factors, thereby controlling transcription of target genes. Preferably, the term refers to either a protein or protein complex transcriptional factor triggered by the cleavage of one of the respective signaling pathway proteins resulting in an intracellular domain.

The term "target gene", as used herein, means a gene whose transcription is directly or indirectly controlled by a respective transcription factor element. The "target gene" may be a "direct target gene" and/or an "indirect target gene" (as described herein).

Pathway analysis enables quantitative measurement of signal transduction pathway activity in cells in a blood sample, based on inferring activity of a signal transduction pathway from measurements of mRNA levels of the well-validated direct target genes of the transcription factor associated with the respective signaling pathway (see for example [10]; [11]).

The term "sample", or "blood sample" as used herein, also encompasses the case where e.g. a tissue and/or draining lymph node and/or blood of the subject have been taken from the subject and, e.g., have been put on a microscope slide, and where for performing the claimed method a portion of this sample is extracted, e.g., by means of Laser Capture Microdissection (LCM), or by scraping off the cells of interest from the slide, or by fluorescence-activated cell sorting techniques.

In an embodiment, the method further comprises the step of determining the expression levels of the three or more, e.g. three, four, five, six, seven eight, nine, ten, eleven, twelve, thirteen or more, target genes of the Notch cellular signaling pathway and optionally the three or more, e.g. three, four, five, six, seven eight, nine, ten, eleven, twelve, thirteen or more, target genes of the JAK-STAT3 cellular signaling pathway and/or further comprises the step of providing or obtaining the blood sample from the subject.

Methods to collect and store blood, and optionally to separate the blood sample in separate fractions or specific cell types, are generally known in the field and also described above. Upon providing the blood sample it can be processed immediately or it can be stored for later processing, e.g. at appropriate storage conditions at 4 °C, -20 °C or -80 °C depending on the intended storage time. When processing the mRNA, it is extracted from the sample using known methods and preferably normalized, e.g. based on the total RNA concentration. Subsequently the extracted mRNA is used to determine the expression levels of the target genes by known methods, such as qPCR, multiple qPCR, ddPCR, RNAseq and/or RNA expression array.

Preferably the determining of the activity or activities of the signaling pathway(s), the combination of multiple pathway activities and applications thereof is performed as described for example in the following documents, each of which is hereby incorporated in its entirety for the purposes of determining activity of the respective signaling pathway: published international patent applications WO2013011479 (titled "ASSESSMENT OF CELLULAR SIGNALING PATHWAY ACTIVITY USING PROBABILISTIC MODELING OF TARGET GENE EXPRESSION"), WO2014102668 (titled "ASSESSMENT OF CELLULAR SIGNALING PATHWAY ACTIVITY USING LINEAR COMBINATION(S) OF TARGET GENE EXPRESSIONS"), WO2015101635 (titled "ASSESSMENT OF THE PI3K CELLULAR SIGNALING PATHWAY ACTIVITY USING MATHEMATICAL MODELLING OF TARGET GENE EXPRESSION"), WO2016062891 (titled "ASSESSMENT OF TGF-β CELLULAR SIGNALING PATHWAY ACTIVITY USING MATHEMATICAL MODELLING OF TARGET GENE EXPRESSION"), WO2017029215 (titled "ASSESSMENT OF NFKB CELLULAR SIGNALING PATHWAY ACTIVITY USING MATHEMATICAL MODELLING OF TARGET GENE EXPRESSION"), WO2014174003 (titled "MEDICAL PROGNOSIS AND PREDICTION OF TREATMENT RESPONSE USING MULTIPLE CELLULAR SIGNALLING PATHWAY ACTIVITIES"), WO2016062892 (titled "MEDICAL PROGNOSIS AND PREDICTION OF TREATMENT RESPONSE USING MULTIPLE CELLULAR SIGNALING PATHWAY ACTIVITIES"), WO2016062893 (titled "MEDICAL PROGNOSIS AND PREDICTION OF TREATMENT RESPONSE USING MULTIPLE CELLULAR SIGNALING PATHWAY ACTIVITIES"), WO2018096076 (titled "Method to distinguish tumor suppressive FOXO activity from oxidative stress"), and in the patent applications WO2018096076 (titled "Method to distinguish tumor suppressive FOXO activity from oxidative stress"), WO2019068585 (titled "Assessment of Notch cellular signaling pathway activity using mathematical modelling of target gene expression"), WO2019120658 (titled "Assessment of MAPK-MAPK-AP1 cellular signaling pathway activity using mathematical modelling of target gene expression"), WO2019068543 (titled "Assessment of JAK-JAK-STAT3 cellular signaling pathway activity using mathematical modelling of target gene expression"), WO2019068562 (titled "Assessment of JAK-STAT1/2 cellular signaling pathway activity using mathematical modelling of target gene expression"), and WO2019068623 (titled "Determining functional status of immune cells types and immune response").

The models have been biologically validated for ER, AR, PI3K-FOXO, HH, Notch, TGF-β, Wnt, NFkB, JAK-STAT1/2, JAK-STAT3 and MAPK-AP1 pathways on several cell types.

Unique sets of cellular signaling pathway target genes whose expression levels are preferably analyzed have been identified. For use in the mathematical models, three or more, for example, three, four, five, six, seven, eight, nine, ten, eleven, twelve or more, target genes from each assessed cellular signaling pathway can be analyzed to determine pathway activities.

Common to the pathway analysis methods for determining the activities of the different signaling pathways as disclosed herein is a concept, which is preferably applied herein for the purposes of the present invention, wherein the activity of a signaling pathway in a cell such as a cell present in a blood sample is determinable by receiving expression levels of one or more, preferably three or more, target genes of the signaling pathway, determining an activity level of a signaling pathway associated transcription factor (TF) element in the sample, the TF element controlling transcription of the three or more target genes, the determining being based on evaluating a calibrated mathematical pathway model relating expression levels of the one or more, preferably three or more target genes to the activity level of the signaling pathway, and optionally inferring the activity of the signaling pathway in the cell present in a blood sample based on the determined activity level of the signaling pathway associated TF element. As described herein, the activity level can be directly used as an input to determine the immunosuppressive state in a subject and/or determine whether a subject is resistant to immune checkpoint inhibitors and/or to distinguish between different types of CD4+ T cells, which is also contemplated by the present invention.

The term "activity level" of a TF element, as used herein, denotes the level of activity of the TF element regarding transcription of its target genes.

The calibrated mathematical pathway model may be a probabilistic model, preferably a Bayesian network model, based on conditional probabilities relating the activity level of the signaling pathway associated TF element and the expression levels of the three or more target genes, or the calibrated mathematical pathway model may be based on one or more linear combination(s) of the expression levels of the three or more target genes. For the purposes of the present invention, the calibrated mathematical pathway model is preferably a centroid or a linear model, or a Bayesian network model based on conditional probabilities.

In particular, the determination of the expression level and optionally the inferring of the activity of a signaling pathway in the subject may be performed, for example, by inter alia (i) evaluating a portion of a calibrated probabilistic pathway model, preferably a Bayesian network, representing the cellular signaling pathways for a set of inputs including the expression levels of the three or more target genes of the cellular signaling pathway measured in a sample of the subject, (ii) estimating an activity level in the subject of a signaling pathway associated transcription factor (TF) element, the signaling pathway associated TF element controlling transcription of the three or more target genes of the cellular signaling pathway, the estimating being based on conditional probabilities relating the activity level of the signaling pathway associated TF element and the expression levels of the three or more target genes of the cellular signaling pathway measured in the sample of the subject, and optionally (iii) inferring the activity of the cellular signaling pathway based on the estimated activity level of the signaling pathway associated TF element in the sample of the subject. This is described in detail in the published international patent application WO 2013/011479 A2 ("Assessment of cellular signaling pathway activity using probabilistic modeling of target gene expression"), the contents of which are herewith incorporated in their entirety.

In an exemplary alternative, the determination of the expression level and optionally the inferring of the activity of a cellular signaling pathway in the subject may be performed by inter alia (i) determining an activity level of a signaling pathway associated transcription factor (TF) element in the sample of the subject, the signaling pathway associated TF element controlling transcription of the three or more target genes of the cellular signaling pathway, the determining being based on evaluating a calibrated mathematical pathway model relating expression levels of the three or more target genes of the cellular signaling pathway to the activity level of the signaling pathway associated TF element, the mathematical pathway model being based on one or more linear combination(s) of expression levels of the three or more target genes, and optionally (ii) inferring the activity of the cellular signaling pathway in the subject based on the determined activity level of the signaling pathway associated TF element in the sample of the subject. This is described in detail in the published international patent application WO 2014/102668 A2 ("Assessment of cellular signaling pathway activity using linear combination(s) of target gene expressions").

Further details regarding the inferring of cellular signaling pathway activity using mathematical modeling of target gene expression can be found in W Verhaegh et al., "Selection of personalized patient therapy through the use of knowledge-based computational models that identify tumor-driving signal transduction pathways", Cancer Research, Vol. 74, No. 11, 2014, pages 2936 to 2945.

Herein, the term "Notch transcription factor element" or "Notch TF element" or "TF element" is defined to be a protein complex containing at least the intracellular domain of one of the Notch proteins (Notch1, Notch2, Notch3 and Notch4, with corresponding intracellular domains N1ICD, N2ICD, N3ICD and N4ICD), with a co-factor, such as the DNA-binding transcription factor CSL (CBF1/RBP-JK, SU(H) and LAG-1), which is capable of binding to specific DNA sequences, and preferably one co-activator protein from the mastermind- like (MAML) family (MAML1, MAML2 and MAML3), which is required to activate transcription, thereby controlling transcription of target genes. Preferably, the term refers to either a protein or protein complex transcriptional factor triggered by the cleavage of one of the Notch proteins (Notch1, Notch2, Notch3 and Notch4) resulting in a Notch intracellular domain (N1ICD, N2ICD, N3ICD and N4ICD). For example, it is known that DSL ligands (DLL1, DLL3, DLL4, Jagged1 and Jagged2) expressed on neighboring cells, bind to the extracellular domain of the Notch protein/receptor, initiating the intracellular Notch signaling pathway and that the Notch intracellular domain participates in the Notch signaling cascade which controls expression.

Herein, the term "JAK-STAT1/2 transcription factor element" or "JAK-STAT1/2 TF element" or "TF element" when referring to the JAK-STAT1/2 is defined to be a protein complex containing at least a STAT1-STAT2 heterodimer or a STAT1 homodimer, which is capable of binding to specific DNA sequences, preferably the ISRE (binding motif AGTTTC NTTCNC/T) or GAS (binding motif TTC/A NNG/TAA) response elements, respectively, thereby controlling transcription of target genes. Preferably, the term refers to either a protein or protein complex transcriptional factor that is formed by different stimuli such as IFNs triggered by the binding of the stimulating ligand to its receptor resulting in downstream signaling.

Herein, the term "JAK-STAT3 transcription factor element" or "JAK-STAT3 TF element" or "TF element" when referring to the JAK-STAT3 pathway is defined to be a protein complex containing at least a STAT3 homodimer, which is capable of binding to specific DNA sequences, preferably the response elements with binding motif CTGGGAA, thereby controlling transcription of target genes. Preferably, the term refers to either a protein or protein complex transcriptional factor triggered by the binding of STAT3 inducing ligands such as interleukin-6 (IL-6) and IL-6 family cytokines to its receptor or an intermediate downstream signaling agent between the binding the ligand to its receptor and the final transcriptional factor protein or protein complex.

Herein, the term "TGFbeta transcription factor element" or "TGFbeta TF element" or "TF element" when referring to the TGFbeta pathway is defined to be a protein complex containing at least one or, preferably, a dimer of the TGFbeta members (SMAD1 , SMAD2, SMAD3 , SMAD5 and SMAD8 with SMAD4) or a trimer (two proteins from SMAD1, SMAD2, SMAD3, SMAD5 and SMAD8 with SMAD4), which is capable of binding to specific DNA sequences, thereby controlling transcription of target genes. Preferably, the term refers to either a protein or protein complex transcriptional factor triggered by the binding of TGFbeta to its receptor or an intermediate downstream signaling agent between the binding of TGFbeta to its receptor and the final transcriptional factor protein or protein complex. For example, it is known that TGFbeta binds to an extracellular TGFbeta receptor that initiates an intracellular "SMAD" signaling pathway and that one or more SMAD proteins (receptor-regulated or R-SMADs (SMAD1, SMAD2, SMAD 3, SMAD5 and SMAD8) and SMAD4) participate in, and may form a hetero-complex which participates in, the TGFbeta transcription signaling cascade which controls expression.

Herein, an NFkB transcription factor (TF) element is defined to be a protein complex containing at least one or, preferably, a dimer of the NFkB members (NFKB 1 or p50/p105, NFKB2 or p52/p100, RELA or p65, REL, and RELB), which is capable of binding to specific DNA sequences, thereby controlling transcription of target genes.

Herein, a FOXO transcription factor (TF) element is defined to be a protein complex containing at least one of the FOXO TF family members, i.e., FOXO1, FOXO3A, FOXO4 and FOXO6, which is capable of binding to specific DNA sequences, thereby controlling transcription of target genes.

Therefore, in an embodiment of the first aspect of the invention:
the Notch cellular signaling pathway is inferred based on the determined expression levels of three or more target genes, e.g. three, four, five, six, seven, eight, nine, ten, eleven or twelve or more, selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more e.g. two, three, four, five, six, seven, eight, Notch target genes are selected from the group consisting of: DTX1, HES1, HES4, HES5, HEY2, MYC, NRARP, and PTCRA, and one or more, e.g. one, two, three, four, five, six, seven, eight, nine, ten, eleven or twelve or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC; and/or
the JAK-STAT3 cellular signaling pathway is inferred based on the determined expression levels of three or more, e.g. three, four, five, six, seven, eight, nine, ten, eleven or twelve or more, target genes selected from the group consisting of: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIF1A, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1.

In an embodiment the immunosuppressed state in a subject is indicative of a resistance against immune checkpoint inhibitors as a treatment option for treating the tumor in the subject. Active Treg cells are known to contribute to a tumor induced immunosuppressive state, where subjects have or develop immune checkpoint inhibitor resistance. Therefore, detecting the presence of activated Treg cells among the cells of a blood sample obtained from a subject having a tumor is indicative that the subject is in the immunosuppressive state and thus resistant to immune checkpoint inhibitors. Therefore based on this finding alternative treatment solutions (e.g. non-immune checkpoint inhibitor based treatments) should be considered for this patient.

In an embodiment the absence of an immunosuppressed state in a subject is indicative for the use of immune checkpoint inhibitors as a viable treatment option for treating the tumor in the subject. When no activated Treg cells are detected among the cells of the blood sample of a subject having a tumor, the subject is assumed not to be in the immunosuppressive state, meaning the subject is considered not resistant to immune checkpoint inhibitors, meaning the subject is likely to demonstrate an immune response to the tumor upon administration of immune checkpoint inhibitors. Therefore the absence of activated Treg cells provides a good indication that immune checkpoint inhibitors are a viable treatment option.

It is further envisioned that by performing on a regular interval the method described herein on cells from a blood sample obtained from a subject having a tumor, which subject is undergoing treatment with an immune checkpoint inhibitor, may provide valuable information on the response of the subject to the treatment, particularly with respect to the development of resistance to the immune checkpoint inhibitor.

Therefore, in a second aspect the invention relates to a method for monitoring a treatment response for a subject having a tumor, the subject receiving checkpoint inhibitors, wherein the method comprises diagnosing the immunosuppressive state of the subject as described in the first aspect of the invention,
preferably the method further comprising changing the treatment of the subject from treatment with checkpoint inhibitors to treatment with an alternative treatment option once the subject is diagnosed to be in the immunosuppressive state. Monitoring the treatment response to immune checkpoint inhibitors in a subject may be performed for example daily, weekly, biweekly, monthly, by monthly, quarterly or yearly. Doing so may provide valuable information about the immunosuppressive state of the subject and may be used in assisting in the decision making of the treatment provided to the subject, e.g. in deciding to change the treatment when the patient is found in an immunosuppressive state and thus to have developed resistance to immune checkpoint inhibitors.

It is further envisioned that the methods described herein are part of method of treatment or use of immune checkpoint inhibitors, wherein the immune checkpoint inhibitors are administrated only when the subject is not found to be in an immunosuppressive state, and thus no resistance to immune checkpoint inhibitors has been developed.

Therefore, in a third aspect, the invention relates to an immune checkpoint inhibitor for use in the treatment of cancer in a subject diagnosed to be not in an immunosuppressed state. In an embodiment of the third aspect of the invention the treatment comprises:
- performing the in vitro or ex vivo diagnostic method as described in any one of the first or the second aspect of the invention;
- administering the checkpoint inhibitor to a subject suffering from cancer when the subject is diagnosed to be not in an immunosuppressed state.

In an embodiment of the third aspect of the invention the immune checkpoint inhibitor is selected from the group consisting of PD-1 inhibitors, PD-L1 inhibitors, CTLA-4 inhibitors, LAG3 inhibitors, TIM-3 inhibitors, TIGIT inhibitors and VISTA inhibitors, preferably wherein the immune checkpoint inhibitor is selected from the group consisting of, Nivolumab (PD-1), Pembrolizumab (PD-1), Spartalizumab (PD-1), Cemiplimab (PD-1), Atezolizumab (PD-L1), Avelumab (PD-L1), Durvalumab (PD-L1), Ipilimumab (CTLA-4), relatlimab (LAG3), GSK2831781 (LAG3), LY3321367 (TIM-3), MBG453 (TIM-3), TSR-022 (TIM-3), MTIG7192A (TIGIT), RG6058 (TIGIT), bms-986207 (TIGIT) and JNJ-61610588 (VISTA). Checkpoint inhibitor therapy is a form of cancer immunotherapy. The therapy targets immune checkpoints, key regulators of the immune system that when stimulated can dampen the immune response to an immunologic stimulus. Some cancers can protect themselves from attack by stimulating immune checkpoint targets. Checkpoint therapy can block inhibitory checkpoints, restoring immune system function. Currently approved checkpoint inhibitors target the molecules CTLA4, PD-1, and PD-L1, but other targets such as LAG3, TIM-3, TIGIT and VISTA are currently being investigated as potential targets, with several inhibitors for these targets currently in clinical trials.

In an embodiment of the third aspect of the invention the cancer is selected from the group consisting of: breast cancer, bladder cancer, cervical cancer, colon cancer, esophageal cancer, head and neck cancer, Hodgkin lymphoma, kidney cancer, Leukemia, liver cancer, lung cancer, lymphoma, melanoma, prostate cancer, renal cell cancer, skin cancer, stomach cancer, rectal cancer or a solid tumor.

In an embodiment of the third aspect of the invention the patient is diagnosed using a kit of parts comprising primers and probes for detecting the expression levels of three or more target genes of the Notch and JAK-STAT3 cellular signaling pathways, the three or more, e.g. three, four, five, six, seven, eight, nine, ten, eleven, twelve or more, Notch target genes are selected from the group consisting of:
CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, e.g. two, three, four, five, six, seven, eight, Notch target genes are selected from the group consisting of: DTX1, HES1, HES4, HES5, HEY2, MYC, NRARP, and PTCRA, and one or more, e.g. one, two, three, four, five, six, seven, eight, nine, ten, eleven, twelve or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more, e.g. three, four, five, six, seven, eight, nine, ten, eleven, twelve or more, JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIFIA, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1. Alternatively is disclosed a method of treating cancer in a subject diagnosed to be not in an immunosuppressed state, the treatment comprising administering an immune checkpoint inhibitor. In an embodiment the treatment comprises:
   - performing the in vitro or ex vivo diagnostic method as described in any one of the first or the second aspect of the invention;
   - administering the checkpoint inhibitor to a subject suffering from cancer when the subject is diagnosed to be not in an immunosuppressed state. In an embodiment the invention the immune checkpoint inhibitor is selected from the group consisting of PD-1 inhibitors, PD-L1 inhibitors, CTLA-4 inhibitors, LAG3 inhibitors, TIM-3 inhibitors, TIGIT inhibitors and VISTA inhibitors, preferably wherein the immune checkpoint inhibitor is selected from the group consisting of, Nivolumab (PD-1), Pembrolizumab (PD-1), Spartalizumab (PD-1), Cemiplimab (PD-1), Atezolizumab (PD-L1), Avelumab (PD-L1), Durvalumab (PD-L1), Ipilimumab (CTLA-4), relatlimab (LAG3), GSK2831781 (LAG3), LY3321367 (TIM-3), MBG453 (TIM-3), TSR-022 (TIM-3), MTIG7192A (TIGIT), RG6058 (TIGIT), bms-986207 (TIGIT) and JNJ-61610588 (VISTA), or combinations thereof. In an embodiment the cancer is selected from the group consisting of: breast cancer, bladder cancer, cervical cancer, colon cancer, esophageal cancer, head and neck cancer, Hodgkin lymphoma, kidney cancer, Leukemia, liver cancer, lung cancer, lymphoma, melanoma, prostate cancer, renal cell cancer, skin cancer, stomach cancer, rectal cancer or a solid tumor. In an embodiment the patient is diagnosed using a kit of parts comprising primers and probes for detecting the expression levels of three or more target genes of the Notch and optionally the JAK-STAT3 cellular signaling pathways, the three or more, e.g. three, four, five, six, seven, eight, nine, ten, eleven, twelve or more, Notch target genes are selected from the group consisting of:
      CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, e.g. two, three, four, five, six, seven, eight, Notch target genes are selected from the group consisting of: DTX1, HES1, HES4, HES5, HEY2, MYC, NRARP, and PTCRA, and one or more, e.g. one, two, three, four, five, six, seven, eight, nine, ten, eleven, twelve or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
      the three or more, e.g. three, four, five, six, seven, eight, nine, ten, eleven, twelve or more, JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIFIA, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1.

It is further envisioned that the invention described herein can be used to characterize T cells, particularly CD4+ T cells. Therefore, in a fourth aspect the invention relates to a method for characterizing a T cell based on inferred cellular signaling pathway activities,
wherein the T cell is determined to be a naive T cell if the inferred pathway activity for FOXO is high, for NFkB is low, for JAK-STAT1/2 is low, for JAK-STAT3 is low, for TGFbeta is intermediate and for Notch is low;
wherein the T cell is determined to be an activated T cell if the inferred pathway activity for FOXO is low, for NFkB is intermediate, for JAK-STAT1/2 is intermediate, for JAK-STAT3 is intermediate, for TGFbeta is low and for Notch is low;
wherein the T cell is determined to be a T-helper 1 cell if the inferred pathway activity for FOXO is low, for NFkB is high, for JAK-STAT1/2 is intermediate, for JAK-STAT3 is intermediate, for TGFbeta is low and for Notch is low;
wherein the T cell is determined to be a T-helper 2 cell if the inferred pathway activity for FOXO is intermediate, for NFkB is low, for JAK-STAT1/2 is low, for JAK-STAT3 is intermediate, for TGFbeta is low and for Notch is low;
wherein the T cell is determined to be a resting state Treg cell if the inferred pathway activity for FOXO is intermediate, for NFkB is low, for JAK-STAT1/2 is intermediate, for JAK-STAT3 is intermediate, for TGFbeta is low and for Notch is intermediate;
wherein the T cell is determined to be an activated Treg cell if the inferred pathway activity for FOXO is high, for NFkB is high, for JAK-STAT1/2 is low, for JAK-STAT3 is high, for TGFbeta is intermediate and for Notch is high.

As indicated by the data represented in Fig. 1, T cells can be distinguished based on the different cellular signaling pathway activities for JAK-STAT1/2, JAK-STAT3, Notch, TGFbeta, NFkB and FOXO. Therefore the method is preferably performed on isolated T cells, more preferably on isolated CD4+ T cells.

In an embodiment the cellular signaling pathway activities are inferred based on the expression levels of three or more target genes for each cellular signaling pathway respectively, wherein:
the three or more, e.g. three, four, five, six, seven, eight, nine, ten, eleven, twelve or more, Notch target genes are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, e.g. two, three, four, five, six, seven, eight, Notch target genes are selected from the group consisting of: DTX1, HES1, HES4, HES5, HEY2, MYC, NRARP, and PTCRA, and one or more, e.g. one, two, three, four, five, six, seven, eight, nine, ten, eleven, twelve or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more, e.g. three, four, five, six, seven, eight, nine, ten, eleven, twelve or more, JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1 preferably, either selected from the group consisting of: BCL2L1, BIRC5, CCND1, CD274, FOS, HIFIA, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1;
the three or more, e.g. three, four, five, six, seven, eight, nine, ten, eleven, twelve or more, PI3K/FOXO target genes are selected from the group consisting of AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXI1, NOS3, PCK1, POMC, PPARGCIA, PRDX3, RBL2, SOD2 and TNFSF10, optionally further comprising at least one, e.g. one, two, three, four, five, six, seven, eight, nine, ten, eleven, twelve or more, target gene(s) selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally further comprising at least one, e.g. three, four, five, six, seven, eight, nine, ten, eleven, twelve or more, target gene(s) selected from the group comprising or consisting of: ATG14, BIRC5, IGFBP1, KLF2, KLF4, MYOD1, PDK4, RAG1, RAG2, SESN1, SIRT1, STK11 and TXNIP, more preferably the three or more, e.g. three, four, five, six, seven, eight, nine, ten, eleven, twelve or more, PI3K/FOXO target genes are selected from the group consisting of AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCK1, POMC, PPARGCIA, PRDX3, RBL2, SOD2 and TNFSF10;
the three or more, e.g. three, four, five, six, seven, eight, nine, ten, eleven, twelve or more, NFkB target genes are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;
the three or more, e.g. three, four, five, six, seven, eight, nine, ten, eleven, twelve or more, JAK-STAT1/2 target genes are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E;
the three or more, e.g. three, four, five, six, seven, eight, nine, ten, eleven, twelve or more, TGFbeta target genes are selected from the group consisting of ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7.

In a fifth aspect, the invention relates to a kit of parts comprising primers and probes for detecting the expression levels of three or more target genes of the Notch and JAK-STAT3 cellular signaling pathways, and optionally for detecting the expression levels of three or more target genes of one or more cellular signaling pathway selected from FOXO, NFkB, JAK-STAT1/2 and TGFbeta, wherein
the three or more, e.g. three, four, five, six, seven, eight, nine, ten, eleven, twelve or more, Notch target genes are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more, e.g. two, three, four, five, six, seven, eight, Notch target genes are selected from the group consisting of: DTX1, HES1, HES4, HES5, HEY2, MYC, NRARP, and PTCRA, and one or more, e.g. one, two, three, four, five, six, seven, eight, nine, ten, eleven, twelve or more, Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more, e.g. three, four, five, six, seven, eight, nine, ten, eleven, twelve or more, JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIFIA, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1;
the three or more, e.g. three, four, five, six, seven, eight, nine, ten, eleven, twelve or more, PI3K/FOXO target genes are selected from the group consisting of AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXI1, NOS3, PCK1, POMC, PPARGCIA, PRDX3, RBL2, SOD2 and TNFSF10, optionally further comprising at least one, e.g. one, two, three, four, five, six, seven, eight, nine, ten, eleven, twelve or more, target gene(s) selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally further comprising at least one, e.g. three, four, five, six, seven, eight, nine, ten, eleven, twelve or more, target gene(s) selected from the group comprising or consisting of: ATG14, BIRC5, IGFBP1, KLF2, KLF4, MYOD1, PDK4, RAG1, RAG2, SESN1, SIRT1, STK11 and TXNIP, more preferably the three or more, e.g. three, four, five, six, seven, eight, nine, ten, eleven, twelve or more, PI3K/FOXO target genes are selected from the group consisting of AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCK1, POMC, PPARGCIA, PRDX3, RBL2, SOD2 and TNFSF10;
the three or more, e.g. three, four, five, six, seven, eight, nine, ten, eleven, twelve or more, NFkB target genes are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;
the three or more, e.g. three, four, five, six, seven, eight, nine, ten, eleven, twelve or more, JAK-STAT1/2 target genes are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E;
the three or more, e.g. three, four, five, six, seven, eight, nine, ten, eleven, twelve or more, TGFbeta target genes are selected from the group consisting of ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7.

Such kit is beneficial as it can be used for the methods and uses described herein.

Particularly, in a sixth aspect the invention relates to use of the kit according to the fifth aspect of the invention in the *in vitro* or *ex vivo* diagnosis of an immunosuppressed state in a subject having a tumor, wherein the diagnosis is based on cells a blood sample. In an embodiment, the cells in the blood sample are T cells, preferably wherein the cells in the blood sample are isolated T cells, more preferably wherein the cells in the blood sample are isolated CD4+ T cells. In a preferred aspect the kit is used in a method or use according to the first, second, third or fourth aspect of the invention.

Further advantages will be apparent to those of ordinary skill in the art upon reading and understanding the attached figures, the following description and, in particular, upon reading the detailed examples provided herein below.

This application describes several preferred embodiments. Modifications and alterations may occur to others upon reading and understanding the preceding detailed description. It is intended that the application is construed as including all such modifications and alterations insofar as they come within the scope of the appended claims or the equivalents thereof.

Other variations to the disclosed embodiments can be understood and effected by those skilled in the art in practicing the claimed invention, from a study of the drawings, the disclosure, and the appended claims.

It shall be understood that the methods of the first and second aspect, the use of the third aspect, the method of the fourth aspect, the kit of the fifth aspect, and use of the kit of the sixth aspect have similar and/or identical preferred embodiments, in particular, as defined in the dependent claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Calculations like the determination of the immunosuppressive state performed by one or several units or devices can be performed by any other number of units or devices.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

General: In all the figures where signal transduction pathway analysis scores are depicted, these are given as log2odds scores for pathway activity, derived from the probability scores for pathway activity provided by the Bayesian pathway model analysis. Log2odds scores indicate the level of activity of a signaling pathway on a linear scale.

Analyzed public datasets are indicated with their GSE number (inthe figure legend of each figure).

All validation samples for a signaling pathway model or an immune response/system model are independent samples and have not been used for calibration of the respective model to be validated.

Fig. 1. Signal transduction pathway activity in CD4+ T cells. A,B. GSE71566, CD4+ T cells derived from cord blood, resting or activated using anti CD3/CD28 (A), or stimulated with IL-12 and IL-4 for differentiation towards respectively Th1 and Th2 T cells (B) [15]. C. GSE11292, CD4+ Treg cells, resting or activated with anti-CD3/-CD28 with IL-2 [16].

The first column contains the sample annotation as available from GEO database (https://www.ncbi.nlm.nih.gov/geo/); top of columns contains names of signaling pathways; note that FOXO transcription factor activity is presented which is the inverse of PI3K pathway activity. Signaling pathway activity scores are presented on log2odds scale with color coding ranging from blue (most inactive) to red (most active).

Fig. 2. Effect of breast cancer tissue supernatant on signal transduction pathway activity scores in resting and activated CD4+ T cells. Dataset GSE36766, resting or in vitro activated (anti-CD3/CD28) CD4+ T cells from healthy donor blood were incubated with primary cancer tissue supernatant (n=4 different cancers) or control medium (n=3) [17]. The left set of columns contain information on the experimental protocol for each sample. Signaling pathway activity scores are presented on a log2odds scale with color coding ranging from blue (most inactive) to red (most active).

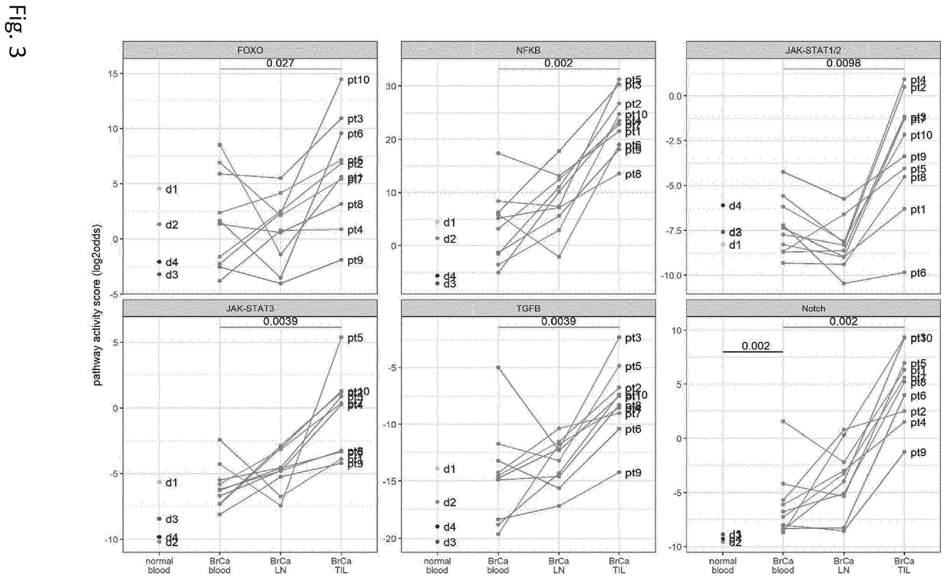

Fig. 3. Signal transduction pathway activity in CD4+ T cells from matched blood, lymph node (LN), and TIL samples of ten patients with invasive breast cancer (BrCa) and four healthy donors; dataset GSE36765 [17]. From left to right: FOXO, NFκB, JAK-STAT1/2, JAK-STAT3, TGFβ, and Notch pathway activity scores. Pathway activity is indicated as log2odds on the y-axis. Individual sample pathway scores are matched by color-coding ('pt' for BrCa patient samples; 'd' for healthy donor samples). P-values (two-sided Wilcoxon-test) are listed when significant (p<0.05); a paired test was performed when comparing amongst BrCa samples.

Fig. 4. GSE36765. Pathway activity in TIL CD4+ T cells, related to the additional clinical information which was described before [17]. Signaling pathway activity scores are presented as log2odds with color coding ranging from blue (most inactive) to red (most active).

Fig. 5. Signaling pathway activities per patient from dataset GSE36765 [17]. Fig. 6. NFκB pathway activity is highly correlated with JAK-STAT3 and TGFβ pathway activity.

Correlation matrix between signaling pathway activities among BrCa TIL samples from dataset GSE36765 [17]. Bottom graphs show bivariate scatterplots with a fitted line and on top are the corresponding Pearson correlation coefficients, including starts for statistically significant correlations.

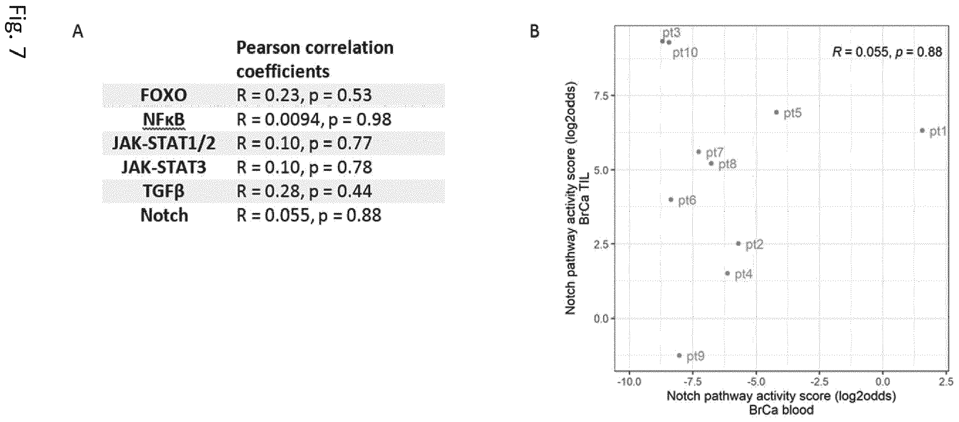

Fig. 7. Correlation between signaling pathway activities in CD4 T+ cells from blood and from TIL from BrCa patients (dataset GSE36765, [17]). A. Pearson correlation coefficients; B. Correlation plot for the Notch pathway.

Fig. 8. Abnormal pathway activities in blood derived CD4+ T cells from ten patients with primary breast cancer (GSE36765, [17]), using normal pathway activity score + 2SD as an upper threshold for normal.

Fig. 9. Threshold values above which signaling pathway activity may be considered abnormally high. Thresholds are calculated based on activity in CD4+ T cells in blood from the four healthy donors in the GSE36765 dataset [17] and are defined as the mean pathway activity score ± two standard deviations.

### EXAMPLES

### Methods

### Signaling pathway activity analysis of preclinical and clinical studies

Tests to quantitatively measure functional activity of PI3K, NFκB, TGFβ, JAK-STAT1/2, JAK-STAT3, and Notch signal transduction pathways on Affymetrix Human Genome U133 Plus 2.0 expression microarrays data have been described before [5], [12], [13], [14]. In brief, the pathway assays are based on the concept of a Bayesian network computational model which calculates from mRNA levels of a selected set, usually between 20 and 30, target genes of the pathway-associated transcription factor a probability score for pathway activity, which is translated to a log2 value of the transcription factor odds, i.e. a log2odds score scaling with pathway activity. The models have all been calibrated on a single cell type and were subsequently frozen and validated on a number of other cell and tissue types without further adaptations of the models. The range (minimum-maximum pathway activity) on the log2odds scale is different for each signaling pathway. While the signaling pathway assays can be used on all cell types, the log2odds score range may vary per cell/tissue type. Of note, measurement of the activity of the PI3K pathway assay is based on the inverse inference of activity of the PI3K pathway from the measured activity of the FOXO transcription factor, in the absence of cellular oxidative stress [12]. For this reason, the FOXO activity score is presented in the figures instead of PI3K pathway activity. In cell culture experiments *in vitro,* PI3K pathway activity can be directly (inversely) inferred from FOXO activity. Activity scores were calculated for the FOXO transcription factor, and NFκB, JAK-STAT1/2, JAK-STAT3, Notch and TGFβ signaling pathways on Affymetrix expression microarray datasets from the Gene Expression Omnibus (GEO, www.ncbi.nlm.nih.gov/geo) database or generated in our own lab, and presented on a log2odds scale.

The GSE71566 dataset contained Affymetrix data from CD4+ T cells isolated from cord blood. Naive CD4+ T cells were compared with CD4+ T cells which had been activated by treatment with anti-CD3 and anti-CD28, and with CD4+ T cells which had been differentiated to either Th1 or Th2 cells [15]. The GSE11292 dataset contained data from cell-sorted Treg cells (CD4+CD25hi), activated *in vitro* with anti-CD3/CD28 in combination with interleukin 2 (IL-2) in a time series [16].

Two Affymetrix datasets have been described in detail before [17] and are also publically available under accession numbers GSE36765 and GSE36766. The GSE36766 dataset contains Affymetrix data from an *in vitro* study in which breast cancer tissue sections from fresh surgical specimens of primary untreated breast cancers (n=4) were mechanically dissociated in X-VIVO 20 medium; CD4+ T cells from healthy donor blood were incubated for 24 hours with anti-CD3/CD28 with or without primary tumor X-VIVO 20 supernatant, and microarray analysis was performed. The GSE36765 dataset is a clinical dataset containing data from patients with primary untreated breast cancer; methods and patient characteristics have been described in detail before [17]. In brief, CD4+ T cells were isolated from primary tumors, axillary lymph nodes, and peripheral blood of ten patients with invasive breast carcinomas, and peripheral blood of four healthy donors.

### Microarray data source and quality control

Analyzed datasets contained Affymetrix Human Genome U133 Plus 2.0 expression microarray data. Quality control (QC) was performed on Affymetrix data of each individual sample based on twelve different quality parameters following Affymetrix recommendations and previously published literature. In summary, these parameters include the average value of all probe intensities, presence of negative or extremely high (> 16-bit) intensity values, poly-A RNA (sample preparation spike-ins) and labelled cRNA (hybridization spike ins) controls, *GAPDH* and *ACTB* 3'/5' ratio, the centre of intensity and values of positive and negative border controls determined by affyQCReport package in R, and an RNA degradation value determined by the AffyRNAdeg function from the Affymetrix package in R. Samples that failed QC were removed prior to data analysis.

### Statistics

Mann-Whitney U testing was used to compare pathway activity scores across groups. In case another statistical method was more appropriate due to the content of a specific dataset, this is indicated in the legend of the figure. For pathway correlation statistics, Pearson correlation tests were performed. Exact p-values are indicated in the figures.

### Defining a threshold for abnormal pathway activity in blood CD4+ T cell samples

To enable clinical use of the pathway activity test on blood samples a preliminary threshold value was calculated above which signaling pathway activity may be considered as abnormally high. The GSE36765 dataset contained blood derived CD4+ T cell samples from four healthy individuals. For each pathway, the mean pathway activity score ± 2SD was calculated and the upper threshold for normal defined as the mean+2SD.

### Results

The activity of the FOXO transcription factor, and of the NFκB, JAK-STAT1/2, JAK-STAT3, Notch, and TGFβ signaling pathways was measured in resting and activated CD4+ T cells, in CD4+ T cells differentiated to Th1 and Th2 cells, in Treg cells, in CD4+ T cells incubated with breast cancer supernatant, and in a series of matched blood, lymph node and tumor infiltrate samples from patients with breast cancer. As mentioned, pathway activity scores are presented on a log2odds scale, with a varying activity range (from minimum to maximum measured pathway activity) on this scale per pathway and cell/tissue type.

### Signaling pathway activity in resting and activated CD4+ T cells, in CD4+ derived Th1 and Th2, and in CD4+ Treg cells

CD4+ T cells can have different phenotypes with specialized functions in the immune response, such as Th1, Th2, and the strongly immune-suppressive Treg cells. We performed signaling pathway analysis on these different CD4+ T cell types generated *in vitro,* and observed very specific signaling pathway activity profiles for the different cell types. In all samples, PI3K pathway activity could be inversely inferred from FOXO activity since there was no evidence for cellular oxidative stress interfering with the inverse relationship between FOXO and PI3K pathway activity (results not shown) [12]. Conventional activation of CD4+ T cells obtained from cord blood with anti-CD3/anti-CD28 antibodies resulted in activation of the NFκB pathway, and slightly increased JAK-STAT1/2 and JAK-STAT3 pathway activity, while the activity of the TGFβ pathway, already low, was further reduced (Fig. 1A). FOXO activity decreased, indicating activation of the PI3K growth factor pathway. Activated T cells which had been further differentiated *in vitro* to respectively Th1 and Th2 T cells, showed differential changes in pathway activity: NFκB and JAK-STAT1/2 pathway activity increased in Th1, while the activity of the FOXO transcription factor increased in Th2 cells (Fig. 1B).

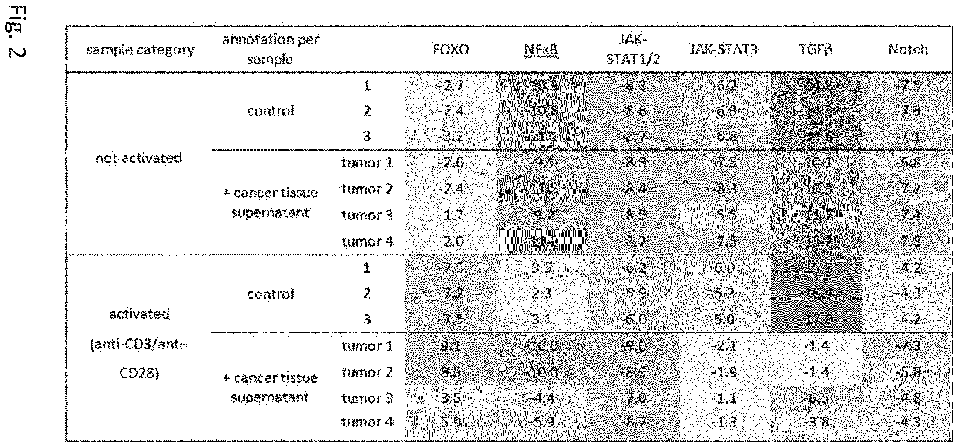

In activated CD4+ T cells from GSE36766, in which CD4+ T cells had been derived from peripheral blood from healthy volunteers and subsequently had been activated by anti-CD3/anti-CD28 incubation, a similar CD4+ T cell pathway activity profile was observed after activation with anti-CD3/CD28 (Fig. 2, compare not activated-control with activated-control) [17].

A distinct type of CD4+ T cells are the Treg cells, which in the analyzed set had been activated with anti-CD3/CD28 plus IL-2. Treg cells were clearly distinguishable from the other CD4+ T cell types. Resting state Treg cells mostly resembled Th2 cells, but with a slightly higher JAK-STAT1/2, lower JAK-STAT3, and higher Notch pathway activity (Fig. 1C). Induction of the Treg immune suppressive state (iTreg cells) was associated with increased FOXO activity, reflecting a reduction in PI3K pathway activity, increased NFκB, JAK-STAT3, TGFβ, and Notch pathway activity, and reduced JAK-STAT1/2 pathway activity (Fig. 1C).

These quantitative pathway analysis results support a mechanistic role for the PI3K-FOXO, NFκB, JAK-STAT1/2 and JAK-STAT3, pathways during activation of CD4+ T cells differentiation to Th1 and Th2 T cells, while the activity of the TGFβ and Notch signaling pathways was specifically linked to the iTreg cells.

### Immune suppressive effect of cancer cell supernatant on activated CD4+ T cells

Using the previously published study in which the effect of adding breast cancer tissue supernatant (SN) to resting or activated CD4+ T cells of healthy individuals was investigated (dataset GSE36766), allowed us to analyze the effect of breast cancer tissue on signaling pathway activity in CD4+ T cells (Fig. 2). While the addition of supernatants of four different dissected primary breast cancer tissue samples did not affect signaling pathway activities in resting CD4+ T cells, the same supernatants changed pathway activity scores in activated CD4+ T cells: NFκB, and JAK-STAT1/2 and JAK-STAT3 pathway activity decreased, while the activity of the TGFβ pathway increased (Fig. 2). In addition, FOXO activity increased, reflecting a decrease in PI3K pathway activity as a consequence of incubation with tumor supernatant. Importantly, after the addition of SN, the JAK-STAT3 pathway activity remained higher than in resting CD4+ T cells, while the activity scores of the FOXO transcription factor increased even above those seen in resting CD4+ T cells. In summary, cancer tissue supernatant caused a partial reversion of the pathway activity profile towards that of resting CD4+ T cells in combination with a strong upregulation of the immune-suppressive TGFβ pathway and decrease in PI3K pathway activity, in line with the induction of an immunotolerant state.

### Clinical study: signaling pathway activity in CD4+ T cells derived from blood, lymph node, and breast cancer tissue samples of patients with primary breast cancer

After characterizing the signaling pathway activity profile associated with activated (anti-CD3/CD28) and immunotolerant CD4+ T cells *in vitro,* CD4+ T cells that had been obtained previously in a clinical patient setting were investigated [17]. Matched CD4+ T cells from peripheral blood, lymph nodes, and tumor infiltrate of ten early untreated breast cancer patients were compared with respect to the above identified signaling pathway activities.

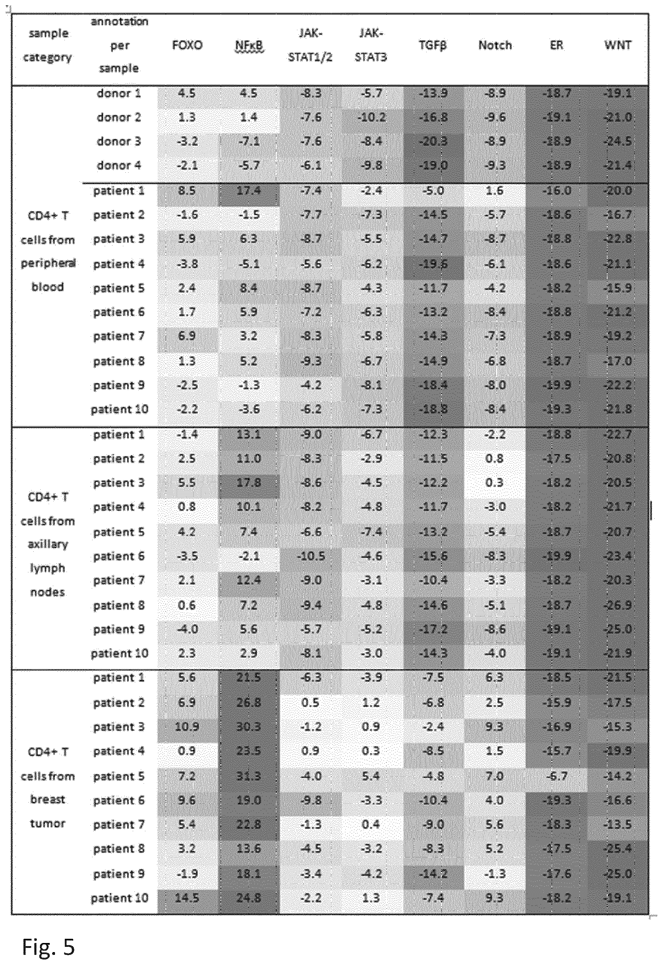

Notch pathway activity was significantly increased (p=0.002) in blood samples from breast cancer patients compared to healthy individuals (Fig. 3). Increased JAK-STAT3, and TGFβ pathway activity scores were measured in a sample subset, but compared to the four control samples this did not reach a statistically significant difference (Fig. 3). In CD4+ T cells isolated from tumor infiltrate (TIL), NFκB, JAK-STAT1/2, JAK-STAT3, TGFβ, and Notch pathway activity were significantly increased compared to the pathway activity scores in corresponding patient blood samples. FOXO activity was also increased in TIL, indicative of an inactivated PI3K pathway (Fig. 3; Fig. 5). Comparing absolute pathway activity scores of TIL samples with the pathway scores measured in the *in vitro* cancer supernatant study, TIL-derived CD4+ T cells mostly resembled the *in vitro* activated CD4+ T cells that had been incubated with cancer tissue supernatant (Figs. 2,3). To identify which subset of CD4+ T cells was responsible for the observed pathway profile in the TIL, this was compared with the *in vitro* identified profiles. The TIL-derived CD4+ T cells profile was in the majority of patients highly similar to the iTreg cell profile, characterized by low PI3K pathway activity, intermediate TGFbeta and high JAK-STAT3, NFκB and Notch pathway activity.

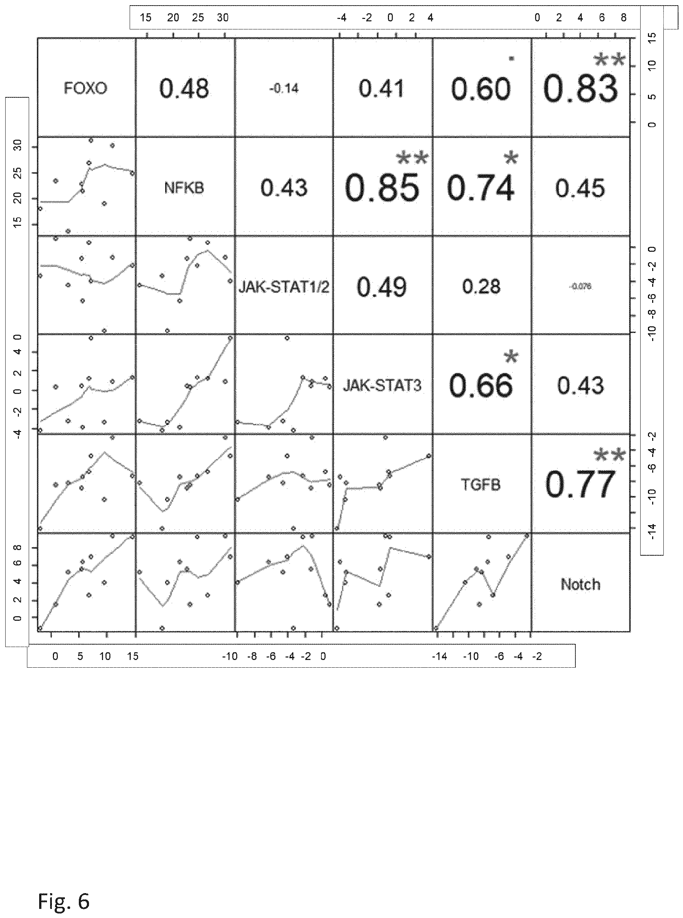

The activity of several signaling pathways was strongly correlated in TIL samples, suggesting that they were often combined active in the same CD4+ T cells (Fig. 6).

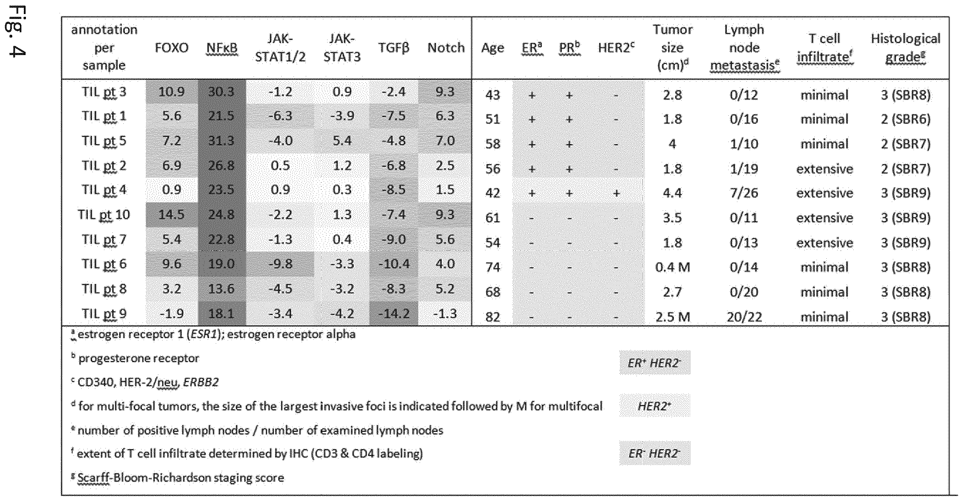

Compared to the *in vitro* T cell experiments, in the clinical samples pathway activity scores varied more between patients, reflecting heterogeneity of the analyzed breast cancer patient group that consisted of four ER/PR positive luminal patients, one ER/PR/HER2 positive patient, and five triple negative patients [17] (Fig. 4; Fig. 5). Although the number of patients per subtype was too small to draw any conclusions on subtype specific pathway profiles in CD4+ TIL cells, it is interesting to note that TGFβ and NFκB pathway activity, were significantly lower in triple negative patients compared to other subtypes (p=0.028 and p=0.028 respectively).

### A threshold for abnormal pathway activity in blood CD4+ T cell samples

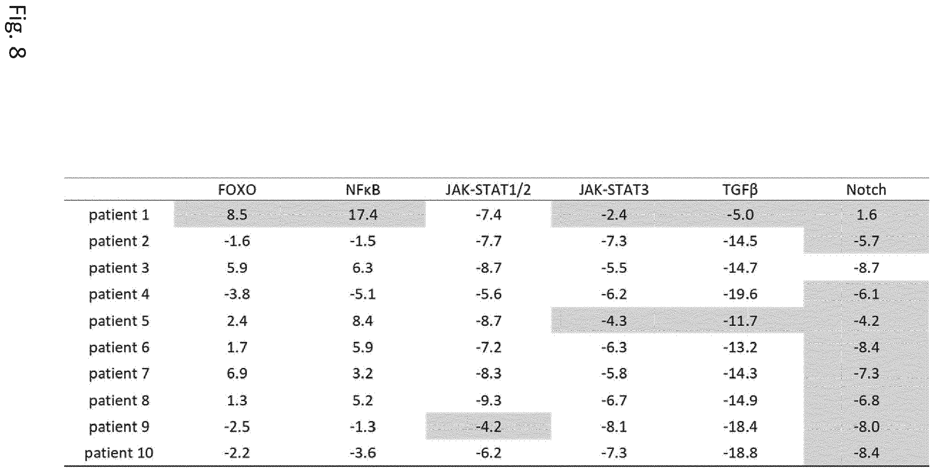

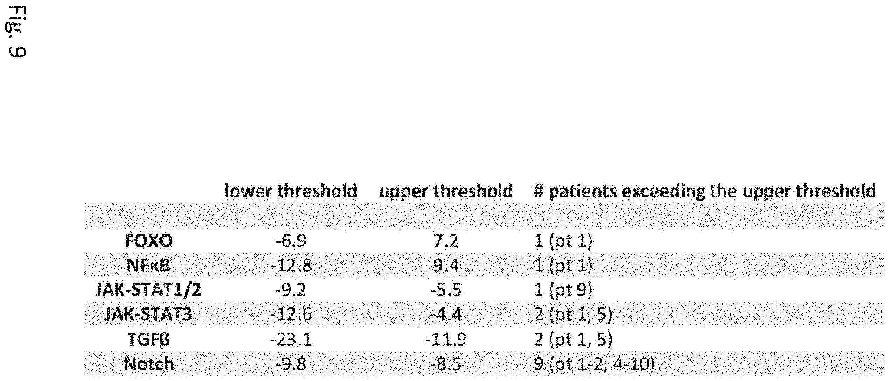

For each pathway, the mean pathway activity score ± 2 standard deviations (SD) was calculated and the upper threshold for normal defined as the mean + 2SD (Fig. 9). When applying these thresholds to the patient samples, combined Notch, JAK-STAT3, and TGFβ pathway activity was identified in 2 patients, abnormal Notch pathway activity in 9 out of 10 patients (Fig. 8 and Fig. 9).

### Discussion

The recently developed assay platform for measuring the activity of the most relevant signal transduction pathways was used to *in vitro* characterize the functional state of different subsets of CD4+ T cells with respect to signaling pathway activity, identify the pathway profile of immunotolerant CD4+ T cells, and analyze the immunosuppressive effect of cancer tissue supernatant. Subsequently, the comparison was made with CD4+ T cell samples from breast cancer patients to identify the CD4+ T cell subset responsible for immunotolerance in TIL, and to investigate whether the immunotolerant CD4+ T cell pathway profile can also be detected in blood samples from breast cancer patients. The signal transduction pathway assays used in the current study have been biologically validated on multiple cell types, including immune cell types. Affymetrix expression microarray data were used for the signaling pathway analysis, however, it is good to keep in mind that with this assay technology Affymetrix microarrays are only used as a method to measure a carefully preselected set of mRNA levels, and not to discover a new gene profile. From Affymetrix whole transcriptome data, expression data of only 20-30 pathway target genes per signaling pathway are used to calculate a signaling pathway activity score, the respective genes have been described.

The measured changes in pathway activity scores associated with CD4+ T cell activation were consistent across two independent studies [17], [15]. Increased PI3K (inferred from a decrease in FOXO activity), NFκB, JAK-STAT1/2, and JAK-STAT3 pathway activity reflects T cell activation, and is of crucial importance for clonal proliferation and execution of specific immune response functions. Differentiation of activated CD4+ T cells to Th1, Th2 and Treg cells appeared to be associated with characteristic alterations in the pathway activity profile, reflecting specific cell functions determined by signaling pathway. For example, In Th1 compared to Th2 cells, a higher JAK-STAT1/2 pathway activity is necessary for the Th1 role to activate CD8+ T cells (Th1 cells have intermediate JAK-STAT1/2 activity, Th2 cells have low JAK-STAT1/2 activity), for example in viral infections, while a high PI3K pathway activity is required for expression of the TBET transcription factor, essential for Th1 function. In immune suppressive iTreg cells, the identified combined activity of Notch with JAK-STAT3 (both high) and TGFβ (intermediate) pathways is in line with the Notch pathway controlling the immune suppressive function in cooperation with the other signaling pathways. The very low PI3K pathway activity (measured as high FOXO activity) reflects the minimal role for this signaling pathway in Treg cells.

Cancer tissue supernatant appeared to induce a partial reversal of the CD4+ activation profile *in vitro,* resulting in a profile which was very similar to that described for immunotolerant lymphocytes and suggesting that one or more soluble factors from cancer tissue had induced immunotolerance. An earlier analysis of this study using conventional bioinformatics approaches had resulted in a similar conclusion as to a tumor suppressive effect of cancer supernatant, but without linking this to signaling pathway activity [17]. Candidates capable of inducing the identified pathway activity profile are a soluble form of PD-L1, TGFβ, and IL-6. The PD-L1 receptor PD-1 mediates tolerance induction in part by inhibiting activation of PI3K, explaining the observed inhibition of the PI3K pathway. TGFβ is locally produced and can directly activate the tumor suppressive TGFβ pathway. Interleukin 6 (IL-6) induces activation of the JAK-STAT3 pathway. Crosstalk between these signaling pathways may be required to induce full T cell tolerance.

Following *in vitro* CD4+ T cell subset analysis, CD4+ T cells from blood, lymphocyte, and TIL samples from patients with untreated primary breast cancer were analyzed. Variation in pathway activity scores between patients was larger in these clinical samples, probably caused by variable immune responses determined by factors such as antigenicity of the tumor and genetic variations.

A subset of TIL-derived samples had a pathway activity profile resembling the immune tolerant pathway profile found in the tumor supernatant-treated cells with respect to PI3K, JAK-STAT3, and TGFβ pathway activity. However, TIL samples had higher Notch, JAK-STAT1/2, and NFκB pathway activity scores. Comparison of TIL samples with Th and Treg subset pathway profiles revealed a strong match with iTreg cells, including Treg-specific Notch pathway activity. The higher JAK-STAT1/2 and NFκB pathway activities in TIL compared to *in vitro* iTreg cells may be due to local inflammatory conditions in cancer tissue. Treg cells induce immune suppression in cancer tissue and are thought to mediate resistance against checkpoint inhibitors.

In patient blood CD4+ T cells, Notch pathway activity was significantly increased compared to healthy controls, similarly indicative of the presence of immune suppressive Treg cells. JAK-STAT3 and TGFβ pathway activity were also higher in patient blood samples but this did not reach significance, which can be explained by a mixture of CD4+ T cell subsets being present in blood and a smaller fraction of activated Treg cells than in the TIL. Using bioinformatics analysis, in the earlier reported analysis no significant differences in the transcriptome had been identified between CD4+ T cells from healthy and cancer patients [17]. Without wishing to be bound by theory, current findings may be explained by a Treg subset of CD4+ T cells having cycled from cancer tissue into blood; alternatively, elevated levels of circulating cytokines like IL-6, IL-10 and TGFβ may explain the tumor suppressive profile in these blood samples [6].

It is of clinical relevance that already in primary untreated breast cancer patients activated immune suppressive Treg cells pathway can be present in peripheral blood. While Treg cells can be identified and isolated using flow cytometry and FACS techniques, our pathway analysis enables assessment of resting versus activated functional state. The first clinical study is in progress to investigate whether measuring this activated Treg profile in blood may help to predict resistance to checkpoint inhibitor therapy. For Affymetrix-based signaling pathway analysis, the here defined (preliminary) thresholds for abnormal pathway activity will be used; thresholds will be adapted for qPCR-based pathway activity profiling.

### REFERENCES

[1] L. A. Emens, "Breast Cancer Immunotherapy: Facts and Hopes," Clin Cancer Res, vol. 24, no. 3, pp. 511-520, Feb. 2018, doi: 10.1158/1078-0432.CCR-16-3001.
[2] S. Hendry et al., "Assessing Tumor-infiltrating Lymphocytes in Solid Tumors: A Practical Review for Pathologists and Proposal for a Standardized Method From the International Immunooncology Biomarkers Working Group: Part 1: Assessing the Host Immune Response, TILs in Invasive Breast Carcinoma and Ductal Carcinoma In Situ, Metastatic Tumor Deposits and Areas for Further Research," Adv Anat Pathol, vol. 24, no. 5, pp. 235-251, Sep. 2017, doi: 10.1097/PAP.0000000000000162.
[3] S. Hendry et al., "Assessing Tumor-Infiltrating Lymphocytes in Solid Tumors: A Practical Review for Pathologists and Proposal for a Standardized Method from the International Immuno-Oncology Biomarkers Working Group: Part 2: TILs in Melanoma, Gastrointestinal Tract Carcinomas, Non-Small Cell Lung Carcinoma and Mesothelioma, Endometrial and Ovarian Carcinomas, Squamous Cell Carcinoma of the Head and Neck, Genitourinary Carcinomas, and Primary Brain Tumors," Adv Anat Pathol, vol. 24, no. 6, pp. 311-335, Nov. 2017, doi: 10.1097/PAP.0000000000000161.
[4] C. Solinas, M. Aiello, P. De Silva, C. Gu-Trantien, E. Migliori, and K. Willard-Gallo, "Targeting PD-1 in cancer: Biological insights with a focus on breast cancer," Crit. Rev. Oncol. Hematol., vol. 142, pp. 35-43, Jul. 2019, doi: 10.1016/j.critrevonc.2019.07.011.
[5] W. Bouwman, W. Verhaegh, L. Holtzer, and A. van de Stolpe, "Quantitative measurement of activity of JAK-STAT signaling pathways in blood samples and immune cells to predict innate and adaptive cellular immune response to viral infection and accelerate vaccine development.," bioRxiv, p. 2020.05.13.092759, May 2020, doi: 10.1101/2020.05.13.092759.
[6] Y. Ohue and H. Nishikawa, "Regulatory T (Treg) cells in cancer: Can Treg cells be a new therapeutic target?," Cancer Sci, vol. 110, no. 7, pp. 2080-2089, Jul. 2019, doi: 10.1111/cas.14069.
[7] R. Saleh and E. Elkord, "Acquired resistance to cancer immunotherapy: Role of tumor-mediated immunosuppression," Semin. Cancer Biol., Jul. 2019, doi: 10.1016/j.semcancer.2019.07.017.
[8] A. Tanaka and S. Sakaguchi, "Targeting Treg cells in cancer immunotherapy," Eur. J. Immunol., vol. 49, no. 8, pp. 1140-1146, 2019, doi: 10.1002/eji.201847659.
[9] R. Saleh and E. Elkord, "Treg-mediated acquired resistance to immune checkpoint inhibitors" Cancer Letters, vol. 457 pp. 168-179, 2019, doi: 10.1016/j.canlet.2019.05.003
[10] W. Verhaegh et al., "Selection of personalized patient therapy through the use of knowledge-based computational models that identify tumor-driving signal transduction pathways," Cancer Res., vol. 74, no. 11, pp. 2936-2945, Jun. 2014, doi: 10.1158/0008-5472.CAN-13-2515
[11] W Verhaegh, A van de Stolpe, Oncotarget, 2014, 5(14):5196
[12] H. van Ooijen et al., "Assessment of Functional Phosphatidylinositol 3-Kinase Pathway Activity in Cancer Tissue Using Forkhead Box-O Target Gene Expression in a Knowledge-Based Computational Model," Am. J. Pathol., vol. 188, no. 9, pp. 1956-1972, Sep. 2018, doi: 10.1016/j.ajpath.2018.05.020.
[13] A. van de Stolpe, L. Holtzer, H. van Ooijen, M. A. de Inda, and W. Verhaegh, "Enabling precision medicine by unravelling disease pathophysiology: quantifying signal transduction pathway activity across cell and tissue types," Sci Rep, vol. 9, no. 1, p. 1603, Feb. 2019, doi: 10.1038/s41598-018-38179-x.
[14] A. van de Stolpe, "Quantitative Measurement of Functional Activity of the PI3K Signaling Pathway in Cancer," Cancers, vol. 11, no. 3, p. 293, Mar. 2019, doi: 10.3390/cancers11030293.
[15] K. Kanduri et al., "Identification of global regulators of T-helper cell lineage specification," Genome Med, vol. 7, p. 122, Nov. 2015, doi: 10.1186/s13073-015-0237-0.
[16] F. He et al., "PLAU inferred from a correlation network is critical for suppressor function of regulatory T cells," Mol Syst Biol, vol. 8, p. 624, Nov. 2012, doi: 10.1038/msb.2012.56.
[17] C. Gu-Trantien et al., "CD4+ follicular helper T cell infiltration predicts breast cancer survival," J. Clin. Invest., vol. 123, no. 7, pp. 2873-2892, Jul. 2013, doi: 10.1172/JCI67428.

## Claims

1. A method for in vitro or ex vivo diagnosing an immunosuppressed state in a subject having a tumor, based on cells in a blood sample obtained from said subject, the method comprising
- inferring the Notch cellular signaling pathway activity in the blood sample based on the determined expression levels of three or more target genes in the blood sample obtained from the subject, and
- determining the presence of activated Treg cells in the blood sample obtained from the subject based on the inferred Notch cellular signaling pathway activity, and
- diagnosing the subject in an immunosuppressed state when Treg cells which are in the immune suppressive state are determined to be present in the blood sample,
wherein a high inferred Notch cellular signaling pathway activity in the blood sample obtained from the subject is indicative for the presence of activated Treg cells, wherein the subject is diagnosed to have an immunosuppressed state when activated Treg cells are present.

2. Method for in vitro or ex vivo diagnosing according to claim 1, wherein the cells in the blood sample are T cells, preferably wherein the cells in the blood sample are isolated T cells, more preferably wherein the cells in the blood sample are isolated CD4+ T cells.

3. Method for in vitro or ex vivo diagnosing according to any one of the preceding claims, wherein further the JAK-STAT3 cellular signaling pathway is inferred in the cells in the blood sample based on the determined expression levels of three or more target genes, and wherein the presence of Treg cells which are activated is determined based on the combined inferred Notch and JAK-STAT3 cellular signaling pathway activities.

4. Method for in vitro or ex vivo diagnosing according to any one of the preceding claims, wherein:
the Notch cellular signaling pathway is inferred based on the determined expression levels of three or more target genes selected from the group consisting of: CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target genes are selected from the group consisting of: DTX1, HES1, HES4, HES5, HEY2, MYC, NRARP, and PTCRA, and one or more Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC; and/or
the JAK-STAT3 cellular signaling pathway is inferred based on the determined expression levels of three or more target genes selected from the group consisting of: AKT1, BCL2, BCL2L1, BIRC5, CCND1, CD274, CDKN1A, CRP, FGF2, FOS, FSCN1, FSCN2, FSCN3, HIF1A, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIF1A, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1.

5. Method for in vitro or ex vivo diagnosing according to any one of the preceding claims, wherein the immunosuppressed state in a subject is indicative of a resistance against immune checkpoint inhibitors as a treatment option for treating the tumor in the subject.

6. Method for in vitro or ex vivo diagnosing according to any one of the preceding claims, wherein the absence of an immunosuppressed state in a subject is indicative for the use of immune checkpoint inhibitors as a viable treatment option for treating the tumor in the subject.

7. Method for monitoring a treatment response for a subject having a tumor, the subject receiving checkpoint inhibitors, wherein the method comprises diagnosing the immunosuppressive state of the subject as described in any one of claims 1 to 6,
preferably the method further comprising changing the treatment of the subject from treatment with checkpoint inhibitors to treatment with an alternative treatment option once the subject is diagnosed to be in the immunosuppressive state.

8. An immune checkpoint inhibitor for use in the treatment of cancer in a subject diagnosed to be not in an immunosuppressed state.

9. Immune checkpoint inhibitor for use according to claim 8, wherein the treatment comprises:
- performing the in vitro or ex vivo diagnostic method as described in any one of claims 1 to 7;
- administering the checkpoint inhibitor to a subject suffering from cancer when the subject is diagnosed to be not in an immunosuppressed state.

10. Immune checkpoint inhibitor for use according to claim 8 or 9, wherein the immune checkpoint inhibitor is selected from the group consisting of PD-1 inhibitors, PD-L1 inhibitors, CTLA-4 inhibitors, LAG3 inhibitors, TIM-3 inhibitors, TIGIT inhibitors and VISTA inhibitors, preferably wherein the immune checkpoint inhibitor is selected from the group consisting of, Nivolumab (PD-1), Pembrolizumab (PD-1), Spartalizumab (PD-1), Cemiplimab (PD-1), Atezolizumab (PD-L1), Avelumab (PD-L1), Durvalumab (PD-L1), Ipilimumab (CTLA-4), relatlimab (LAG3), GSK2831781 (LAG3), LY3321367 (TIM-3), MBG453 (TIM-3), TSR-022 (TIM-3), MTIG7192A (TIGIT), RG6058 (TIGIT), bms-986207 (TIGIT) and JNJ-61610588 (VISTA).

11. Immune checkpoint inhibitor for use according to any one of claims 8 to 10, wherein the cancer is selected from the group consisting of: breast cancer, bladder cancer, cervical cancer, colon cancer, esophageal cancer, head and neck cancer, Hodgkin lymphoma, kidney cancer, Leukemia, liver cancer, lung cancer, lymphoma, melanoma, Prostate cancer, renal cell cancer, skin cancer, stomach cancer, rectal cancer or a solid tumor.

12. Method for characterizing a T cell based on inferred cellular signaling pathway activities,
wherein the T cell is determined to be a naive T cell if the inferred pathway activity for FOXO is high, for NFkB is low, for JAK-STAT1/2 is low, for JAK-STAT3 is low, for TGFbeta is intermediate and for Notch is low;
wherein the T cell is determined to be an activated T cell if the inferred pathway activity for FOXO is low, for NFkB is intermediate, for JAK-STAT1/2 is intermediate, for JAK-STAT3 is intermediate, for TGFbeta is low and for Notch is low;
wherein the T cell is determined to be a T-helper 1 cell if the inferred pathway activity for FOXO is low, for NFkB is high, for JAK-STAT1/2 is intermediate, for JAK-STAT3 is intermediate, for TGFbeta is low and for Notch is low;
wherein the T cell is determined to be a T-helper 2 cell if the inferred pathway activity for FOXO is intermediate, for NFkB is low, for JAK-STAT1/2 is low, for JAK-STAT3 is intermediate, for TGFbeta is low and for Notch is low;
wherein the T cell is determined to be a resting state Treg cell if the inferred pathway activity for FOXO is intermediate, for NFkB is low, for JAK-STAT1/2 is intermediate, for JAK-STAT3 is intermediate, for TGFbeta is low and for Notch is intermediate;
wherein the T cell is determined to be an activated Treg cell if the inferred pathway activity for FOXO is high, for NFkB is high, for JAK-STAT1/2 is low, for JAK-STAT3 is high, for TGFbeta is intermediate and for Notch is high.

13. Method according to claim 12, wherein the cellular signaling pathway activities are inferred based on the expression levels of three or more target genes for each cellular signaling pathway respectively, wherein:
the three or more Notch target genes are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target genes are selected from the group consisting of: DTX1, HES1, HES4, HES5, HEY2, MYC, NRARP, and PTCRA, and one or more Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1 preferably, either selected from the group consisting of: BCL2L1, BIRC5, CCND1, CD274, FOS, HIF1A, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1;
the three or more PI3K/FOXO target genes are selected from the group consisting of AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXI1, NOS3, PCK1, POMC, PPARGCIA, PRDX3, RBL2, SOD2 and TNFSF10, optionally further comprising at least one target gene selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally further comprising at least one target gene selected from the group comprising or consisting of: ATG14, BIRC5, IGFBP1, KLF2, KLF4, MYOD1, PDK4, RAG1, RAG2, SESN1, SIRT1, STK11 and TXNIP, more preferably the three or more PI3K/FOXO target genes are selected from the group consisting of AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCK1, POMC, PPARGCIA, PRDX3, RBL2, SOD2 and TNFSF10;
the three or more NFkB target genes are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;
the three or more JAK-STAT1/2 target genes are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E;
the three or more TGFbeta target genes are selected from the group consisting of ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7.

14. Kit of parts comprising primers and probes for detecting the expression levels of three or more target genes of the Notch and JAK-STAT3 cellular signaling pathways, and optionally for detecting the expression levels of three or more target genes of one or more cellular signaling pathway selected from FOXO, NFkB, JAK-STAT1/2 and TGFbeta, wherein
the three or more Notch target genes are selected from the group consisting of CD28, CD44, DLGAP5, DTX1, EPHB3, FABP7, GFAP, GIMAP5, HES1, HES4, HES5, HES7, HEY1, HEY2, HEYL, KLF5, MYC, NFKB2, NOX1, NRARP, PBX1, PIN1, PLXND1, PTCRA, SOX9, and TNC, preferably, wherein two or more Notch target genes are selected from the group consisting of: DTX1, HES1, HES4, HES5, HEY2, MYC, NRARP, and PTCRA, and one or more Notch target genes are selected from the group consisting of: CD28, CD44, DLGAP5, EPHB3, FABP7, GFAP, GIMAP5, HES7, HEY1, HEYL, KLF5, NFKB2, NOX1, PBX1, PIN1, PLXND1, SOX9, and TNC;
the three or more JAK-STAT3 target genes are selected from the group consisting of AKT1, BCL2, BCL2L1, BIRC5, CCND1, CD274, CDKNIA, CRP, FGF2, FOS, FSCN1, FSCN2, FSCN3, HIFIA, HSP90AA1, HSP90AB1, HSP90B1, HSPA1A, HSPA1B, ICAM1, IFNG, IL10, JunB, MCL1, MMP1, MMP3, MMP9, MUC1, MYC, NOS2, POU2F1, PTGS2, SAA1, STAT1, TIMP1, TNFRSF1B, TWIST1, VIM and ZEB1, preferably, either selected from the group consisting of: BCL2L1, BIRC5, CCND1, CD274, FOS, HIFIA, HSP90AA1 , HSP90AB1, MMP 1 , and MYC, or selected from the group consisting of: BCL2L1, CD274, FOS, HSP90B1, HSPA1B, ICAM1, IFNG, JunB, PTGS2, STAT1, TNFRSF1B, and ZEB1;
the three or more PI3K/FOXO target genes are selected from the group consisting of AGRP, BCL2L11, BCL6, BNIP3, BTG1, CAT, CAV1, CCND1, CCND2, CCNG2, CDK 1A, CDK 1B, ESR1, FASLG, FBX032, GADD45A, INSR, MXI1, NOS3, PCK1, POMC, PPARGCIA, PRDX3, RBL2, SOD2 and TNFSF10, optionally further comprising at least one target gene selected from the group comprising or consisting of: ATP8A1, C10orf10, CBLB, DDB1, DYRK2, ERBB3, EREG, EXT1, FGFR2, IGF1R, IGFBP1, IGFBP3, LGMN, PPM ID, SEMA3C, SEPP1, SESN1, SLC5A3, SMAD4 and TLE4, optionally further comprising at least one target gene selected from the group comprising or consisting of: ATG14, BIRC5, IGFBP1, KLF2, KLF4, MYOD1, PDK4, RAG1, RAG2, SESN1, SIRT1, STK11 and TXNIP, more preferably the three or more PI3K/FOXO target genes are selected from the group consisting of AGRP, BCL2L11, BCL6, BNTP3, BTG1, CAT, CAV1, CCND1, CCND2, CCNG2, CDKN1A, CDKN1B, ESR1, FASLG, FBX032, GADD45A, INSR, MXI1, NOS3, PCK1, POMC, PPARGCIA, PRDX3, RBL2, SOD2 and TNFSF10;
the three or more NFkB target genes are selected from the group consisting of BCL2L1, BIRC3, CCL2, CCL3, CCL4, CCL5, CCL20, CCL22, CX3CL1, CXCL1, CXCL2, CXCL3, ICAM1, IL1B, IL6, IL8, IRF1, MMP9, NFKB2, NFKBIA, NFKBIE, PTGS2, SELE, STAT5A, TNF, TNFAIP2, TNIP1, TRAF1, and VCAM1;
the three or more JAK-STAT1/2 target genes are selected from the group consisting of BID, GNAZ, IRF1, IRF7, IRF8, IRF9, LGALS1, NCF4, NFAM1, OAS1, PDCD1, RAB36, RBX1, RFPL3, SAMM50, SMARCB1, SSTR3, ST13, STAT1, TRMT1, UFD1L, USP18, ZNRF3, GBP1, TAP1, ISG15, APOL1, IFI6, IFIRM1, CXCL9, APOL2, IFIT2 and LY6E preferably, from the group consisting of: IRF1, IRF7, IRF8, IRF9, OAS1, PDCD1, ST13, STAT1 and USP1, or from the group consisting of GBP1, IRF9, STAT1, TAP1, ISG15, APOL1, IRF1, IRF7, IFI6, IFIRM1, USP18, CXCL9, OAS1, APOL2, IFIT2 and LY6E;
the three or more TGFbeta target genes are selected from the group consisting of ANGPTL4, CDC42EP3, CDKNIA, CDKN2B, CTGF, GADD45A, GADD45B, HMGA2, ID1, IL11, SERPINE1, INPP5D, JUNB, MMP2, MMP9, NKX2-5, OVOL1, PDGFB, PTHLH, SGK1, SKIL, SMAD4, SMAD5, SMAD6, SMAD7, SNAI1, SNAI2, TIMP1, and VEGFA, preferably, from the group consisting of: ANGPTL4, CDC42EP3, CDKNIA, CTGF, GADD45A, GADD45B, HMGA2, ID1, IL11, JUNB, PDGFB, PTHLH, SERPINE1, SGK1, SKIL, SMAD4, SMAD5, SMAD6, SMAD7, SNAI2, VEGFA, more preferably, from the group consisting of: ANGPTL4, CDC42EP3, CDKNIA, CTGF, GADD45B, ID1, IL11, JUNB, SERPINE1, PDGFB, SKIL, SMAD7, SNAI2, and VEGFA, more preferably, from the group consisting of: ANGPTL4, CDC42EP3, ID1, IL11, JUNB, SERPINE1, SKIL, and SMAD7.

15. Use of the kit according to claim 14 in the in vitro or ex vivo diagnosis of an immunosuppressed state in a subject having a tumor, wherein the diagnosis is based on cells a blood sample,
preferably wherein the cells in the blood sample are T cells,
more preferably wherein the cells in the blood sample are isolated T cells,
even more preferably wherein the cells in the blood sample are isolated CD4+ T cells.
